(19) **Europäisches Patentamt / European Patent Office / Office européen des brevets**

(11) **EP 2 002 016 B1**

(12) **EUROPEAN PATENT SPECIFICATION**

(45) Date of publication and mention
of the grant of the patent:
**18.11.2009 Bulletin 2009/47**

(51) Int Cl.:
**C12Q 1/68** *(2006.01)*

(21) Application number: **07732383.0**

(22) Date of filing: **12.04.2007**

(86) International application number:
**PCT/GB2007/001340**

(87) International publication number:
**WO 2007/129000 (15.11.2007 Gazette 2007/46)**

(54) **METHOD FOR DETERMINING COPY NUMBER**

VERFAHREN ZUR BESTIMMUNG DER KOPIENUMMER

PROCÉDÉ POUR DÉTERMINER LE NUMÉRO DE COPIE

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **12.04.2006 US 791754 P**

(43) Date of publication of application:
**17.12.2008 Bulletin 2008/51**

(73) Proprietor: **Medical Research Council**
**20 Park Crescent**
**London W1B 1AL (GB)**

(72) Inventors:
• **THANGAVELU, Maden**
**Cambridge CB2 0XZ (GB)**
• **DEAR, Paul**
**Cambridge CB2 2QH (GB)**
• **RABBITTS, Terence H.**
**Cambridge CB2 2QH (GB)**
• **DASER, Angelica**
**Cambridge CB2 2QH (GB)**
• **BANKIER, Alan Thomas**
**Swavesey, Cambridge CB2 4QN (GB)**
• **KONFORTOV, Bernard Anri**
**Sawston, Cambridge CB2 4DE (GB)**

(74) Representative: **Holliday, Louise Caroline**
**D Young & Co**
**120 Holborn**
**London EC1N 2DY (GB)**

(56) References cited:
**WO-A-99/14376        WO-A-2004/011678**
**WO-A-2006/058395    DE-A1-102004 036 285**

**DE-A1-102005 045 560    DE-A1-102005 059 227**

• KOVACS G ET AL: "Nonhomologous chromatid exchange in hereditary and sporadic renal cell carcinomas." PROCEEDINGS OF THE NATIONAL ACADEMY OF SCIENCES OF THE UNITED STATES OF AMERICA 1 JAN 1991, vol. 88, no. 1, 1 January 1991 (1991-01-01), pages 194-198, XP002462990 ISSN: 0027-8424
• SANJMYATAV JIMSGENE ET AL: "Comparative study of renal cell carcinoma by CGH, multicolor-FISH and conventional cytogenic banding analysis." ONCOLOGY REPORTS NOV 2005, vol. 14, no. 5, November 2005 (2005-11), pages 1183-1187, XP008086999 ISSN: 1021-335X
• PAVLOVICH CHRISTIAN P ET AL: "Patterns of aneuploidy in stage IV clear cell renal cell carcinoma revealed by comparative genomic hybridization and spectral karyotyping." GENES, CHROMOSOMES & CANCER JUL 2003, vol. 37, no. 3, July 2003 (2003-07), pages 252-260, XP002462991 ISSN: 1045-2257
• PRESTI J C JR ET AL: "HISTOPATHOLOGICAL CYTOGENETIC AND MOLECULAR CHARACTERIZATION OF RENAL CORTICAL TUMORS" CANCER RESEARCH, vol. 51, no. 5, 1991, pages 1544-1552, XP002462992 ISSN: 0008-5472
• DASER ANGELIKA ET AL: "Interrogation of genomes by molecular copy-number counting (MCC)." NATURE METHODS JUN 2006, vol. 3, no. 6, June 2006 (2006-06), pages 447-453, XP008084005 ISSN: 1548-7091

**(Cont. next page)**

• LAU ET AL: "Cytogenetic alterations in renal tumors: a study of 38 Southeast Asian patients" CANCER GENETICS AND CYTOGENETICS, ELSEVIER SCIENCE PUBLISHING, NEW YORK, NY, US, vol. 175, no. 1, 10 May 2007 (2007-05-10), pages 1-7, XP022068240 ISSN: 0165-4608

**Description**

FIELD OF INVENTION

The present invention relates to a method for the detection of changes in the copy-number of nucleic acid sequences - such as genomic DNA - and various applications of this method. By way of example, the method may be used for the detection of genomic alterations and the diagnosis of disease - such as cancer. Moreover, a non-reciprocal t(3;5) translocation identified by the method of the present invention is also described.

BACKGROUND TO THE INVENTION

Chromosome alterations (eg. abnormalities) are often associated with genetic disorders, degenerative diseases, and cancer. The deletion or multiplication of copies of whole chromosomes and the deletion or amplifications of chromosomal segments or specific regions are common occurrences in disease - such as cancer (Breast Cancer Res. Treat. 18: Suppl. 1:5-14; Biochim. Biophys. Acta. 1072:33-50). In fact, amplifications and deletions of DNA sequences can be the cause of a disease. For example, proto-oncogenes and tumour-suppressor genes, respectively, are frequently characteristic of tumorigenesis (Cancer Genet. Cytogenet. 49: 203-217). Clearly, the identification and cloning of specific genomic regions associated with disease is crucial both to the study of the disease and in developing better means of diagnosis and prognosis.

Methods for determining the status of individual genomes are therefore required in the post-genome sequencing era to implement the objectives of personalised, preventative and predictive medicine. Diseased (eg. cancer) genomes display many abnormalities while hereditary chromosomal abnormalities are associated with many complex syndromes and disease predispositions. Indeed, the proposed sequencing of a range of whole cancer genomes might be better focussed on those regions where aberrations are located, using methods of scanning genomes for such changes. Neoplasms often have complex cytogenetic aberrations including deletions, amplications and translocations. Whilst leukaemias and sarcomas typically show reciprocal chromosomal translocations (2), epithelial tumours (which comprise greater than 90% of human cancers) are far more heterogeneous (1). Major events in epithelial tumours are chromosomal gain or loss and unbalanced translocations. In addition to this, small cytogenetically invisible abnormalities exist in tumours, for instance those sometimes found in association with chromosomal translocations (3). Particularly well characterized, nonreciprocal chromosomal translocations are the unbalanced der3 t(3;5) in renal cell carcinoma (4,5). These abnormalities associate specifically with non-papillary renal cell carcinoma, in which it occurs at a frequency of at least 15% (6). Elucidation of the der(3)t(3;5) breakpoints of these translocations has been hampered because they are non-reciprocal.

An approach to this problem could be based on the fact that non-reciprocal translocations result in a change in the copy-number of genomic sequences. A number of hybridization-based techniques are available to scan the copy-number within genomes. These methods use genomic DNA (7) as a probe for arrays of BACS or PACS (8, 9) or oligonucleotides (10, 11) representing the human reference sequence or use representation genomic probes (ROMA) (12-14) or SNP arrays (15). Array-based methods, however, lack flexibility since a new array must be created for each set of targets to be examined. Further, these approaches are not always quantitative since the hybridisation signal reflects the copy-number of the particular region of the genome corresponding to the probe. Other approaches based on quantitative PCR require optimisation for each locus evaluated.

DE102004036285 discloses a method to determine the copy number of a chromosome by providing a sample which corresponds to one cell, i.e. one copy of the genome. Several stretches of the genome are amplified using one primer set, and amplified in a second amplification reaction which amplifies distinct fragments (see paragraphs 35 and 40-41) the presence of which is tested (in the example of DE102004036285 the fragments originate from chromosome 2). The number of amplified fragments is compared with the number of amplified fragments in a reference sample.

The present invention relates to improvements in detecting a change in the copy-number of one or more nucleic acid sequences.

SUMMARY OF THE INVENTION

The method described herein (referred to as Molecular Copynumber Counting, or MCC) measures the copy number frequency by analysing the frequency with which amplification of a nucleic acid sequence - such as a genomic marker - occurs at limiting DNA dilution.

The methods have been validated by copy-number scanning the short arm of human chromosome 3 in renal cell carcinoma to locate the breakpoint of a non-reciprocal translocation to within 300bp, allowing it to be cloned. This is the first time the breakpoint of a *de novo* nonreciprocal translocation in renal cell carcinoma has been cloned.

SUMMARY ASPECTS OF THE PRESENT INVENTION

In a first aspect there is provided a method of measuring the copy number frequency of one or more nucleic acid sequences in a sample, comprising the steps of: (a) providing a plurality aliquot(s) of the sample, wherein each aliquot comprises nucleic acid in an amount that is less than one genome per aliquot; (b) amplifying one or more nucleic acid sequences in each of the aliquot(s) in a first amplification reaction; (c) subdividing one or more of the amplified products into replica aliquots and performing a second amplification reaction for one or more nucleic acid sequences in each of the aliquot(s), wherein at least one of the nucleic acid sequences is a test marker and at least one of the nucleic acid sequences is a reference marker: and (d) calculating the copy number by comparing in each of the replica aliquots the number of amplified products obtained for the test marker with the number of amplified products obtained for the reference marker.

In a second aspect there is provided a method of identifying one or more alterations in a sample of nucleic acid, comprising the steps of: (a) measuring the copy number frequency of one or more nucleic acid sequences in a first sample and a second sample according to the method of the first aspect of the present invention; (b) optionally iteratively repeating the method at progressively higher resolutions for each of the samples; and (c) identifying one or more differences in the copy number frequency of one or more nucleic acid sequences in the first and second samples.

In a third aspect there is provided a method of diagnosing a disease in a subject, comprising the steps of: (a) measuring the copy number frequency of one or more nucleic acid sequences in a sample according to the method of the first aspect of the present invention; and (b) comparing the copy number of the one or more nucleic acid sequences with the normal copy number of the one or more nucleic acid sequences; wherein a difference between the copy numbers of the one or more nucleic acid sequences in the sample and the normal copy number of the one or more nucleic acid sequences is indicative that the subject is suffering from the disease.

In a fourth aspect there is provided a method for cloning one or more alterations in a sample of nucleic acid comprising the steps of: (a) identifying one or more alterations in a sample of nucleic acid according to the second aspect of the present invention; and (b) cloning the or one more alterations.

A fifth aspect relates to an isolated nucleic acid encoding a non-reciprocal t(3;5) translocation, wherein chromosome 5q21.3 to co-ordinate 105386443bp is fused to chromosome 3p13 from co-ordinate 74111893bp and wherein the adenine residue at the junction is from either chromosome.

A sixth aspect relates to a method for diagnosing cancer in a subject comprising the step of determining the presence of the non-reciprocal t(3;5) translocation according to any of claims 24-27, wherein the presence of the translocation is indicative that the subject has cancer.

PREFERRED EMBODIMENTS

Preferably, each aliquot in the first amplification reaction comprises about 0.1-0.9 genomes of DNA per amplification reaction.

Preferably, the copy number of the test marker is calculated by manually counting the number of amplification products for the test marker and the reference marker.

Preferably, the copy number of the test marker is calculated using the equation:

$Np=N(1-e^{-z})$ wherein N is the number of aliquots; Z is the average number of amplified products per aliquot; and Np is the number of aliquots which are expected to contain at least one molecule of the nucleic acid according to Poisson distribution.

Preferably, the copy number of the test marker is calculated using the equation: $Z= -\ln(1-Np/N)$ wherein N is the number of aliquots of nucleic acid tested for a given sequence; Np is the number of aliquots that score positive for the nucleic acid.

Preferably, the amplification reactions are performed using PCR.

Preferably, the first amplification reaction is performed using forward and reverse primer pairs.

Preferably, the second amplification reaction is performed using forward-internal and reverse primers.

Preferably, the sample is derived or derivable from the group consisting of chromosome 1, chromosome 2, chromosome 3, chromosome 4, chromosome 5, chromosome 6, chromosome 7, chromosome 8, chromosome 9, chromosome 10, chromosome 11, chromosome 12, chromosome 13, chromosome 14, chromosome 15, chromosome 16, chromosome 17, chromosome 18, chromosome 19, chromosome 20, chromosome 21, chromosome 22, chromosome X and chromosome Y.

Preferably, the concentration of nucleic acid in the sample prior to aliquoting is determined by UV spectrophotometry.

Preferably, the concentration of nucleic acid in the sample prior to aliquoting is determined by amplifying one or more nucleic acids believed to be present at only one copy per haploid genome at two or more different dilutions, wherein the proportion of samples at each dilution found positive for the one or more nucleic acids is used to refine the estimate of the DNA concentration and hence determine the dilution required for the subsequent analysis.

Preferably, 4 nucleic acids are amplified and 6 dilutions are prepared.

Preferably, the samples are or are derived from diseased and non-diseased subjects.

Preferably, a whole chromosome is initially scanned before focusing on one area for further study.

Preferably, the method is initially performed at a resolution of about 2Mb progressively decreasing to about 100 base pairs or less.

Preferably, the alteration is a translocation, an amplification; a duplication or a deletion.

Preferably, if the copy number of one or more nucleic acid sequences in the sample of nucleic acid from the subject is greater than the normal copy number is indicative of a translocation, an amplification or a duplication.

Preferably, if the copy number of one or more nucleic acid sequences in the sample of nucleic acid from the subject is less than the normal copy number is indicative of a deletion.

Preferably, the disease is cancer.

Preferably, the cancer is kidney cancer.

Preferably, the subject is selected from the group consisting of: (i) a subject that is suffering or is suspected to be suffering from the disease; (ii) a subject that is known to be pre-disposed to the disease; (iii) a subject that has been exposed to one or more agents or conditions that are known or are suspected to cause the disease; and (iv) a subject that is in the process of or is suspected to be in the process of developing the disease.

Preferably, the alteration is cloned using inverse PCR.

Preferably, chromosome 5q21.3 comprises the sequence TATACATACATACGGATATATGTATAAAATC.

Preferably, chromosome 3p13 comprises the sequence TAGGGAGTGAAGTAGTGGCCAAGAAAACATGCCAG.

Preferably, the non-reciprocal t(3;5) translocation comprises the sequence TATACATACATACGGATATATGTATAAAATCATAGGGAGTGAAGTAGTGGCCAAGAAA ACATGCCAG.

Preferably, the cancer is renal cell carcinoma.

ADVANTAGES

The present invention has a number of advantages. These advantages will be apparent in the following description.

By way of example, the present invention is advantageous since the method offers effectively unlimited resolution as sequences can be examined at intervals down to a few hundred base pairs and should be similarly applicable to characterisation of amplified regions.

By way of further example, the present invention is advantageous since it only requires a genome database for its operation and libraries of PCR primersto enable the rapid scanning of chromosomal regions or complete genomes.

By way of further example, the present invention is advantageous because any level of resolution can be achieved depending on the chosen density of markers. Therefore, the area of study is readily under control.

By way of further example, the present invention is advantageous because unlike other DNA counting technologies, the methods described herein do not require whole genome amplification or any hybridisation step. This obviates any problems that might arise from region specific genome amplification, incomplete suppression of repeat sequences within the probe and removes any risk of cross-hybridisation, as can occur in short oligo arrays or in amplification of *E. coli* DNA contaminating BAC/PACs for array CGH (9).

By way of further example, the present invention is advantageous because the methods are essentially digital (counting molecular copy-number) which simplifies interpretation of results whereas the micro-array approaches can require computing algorithms for interpretation (23).

By way of further example, the present invention is advantageous because the methods lend themselves to automation, and are amenable to high-throughput, although it is also easily applicable to manual operation and thus has no mandatory requirement for machinery - such as arrayers.

By way of further example, the present invention is advantageous because the method requires minuscule amounts of genomic DNA, being applicable to DNA from as few as tens to hundreds of cells and the DNA does not have to be high molecular weight. The methods will enable hitherto impractical studies - such as the detailed analysis of pre-neoplastic biopsy material from patients, the retrospective analysis of archival tumour samples, or the exploration of genomic variability across different small regions of a tumour.

By way of further example, the present invention is advantageous because the methods described herein should also simplify the analysis of hereditary chromosomal abnormalities that affect copy-number, whether associated with disease or forming part of a normal spectrum of human variation.

DESCRIPTION OF THE FIGURES

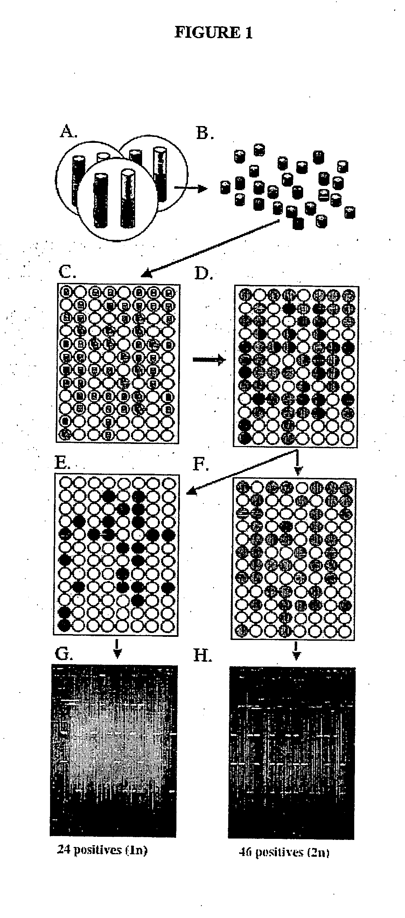

**Figure 1:** *Overview of the Molecular Copy-number Counting method*

Molecular copy-number counting (MCC) relies on the frequency of PCR amplification products from genomic DNA, corresponding to chromosomal markers along a segment of a chromosome. MCC is a two-step procedure. In this figure, (A) cells carry a non-reciprocal translocation and therefore one marker sequence (shown in green) is present in twice as many copies as a second marker (shown in red) that lies telomeric of the translocation breakpoint. (B) DNA is prepared from the target cells and diluted to less than one genome per aliquot. (C) These aliquots are dispensed into wells of a 96-well plate; for simplicity, only the red and green markers are illustrated, showing the restricted number of wells receiving red and green marker genomic DNA. (D) An initial multiplex PCR amplification is conducted to amplify all markers by a modest amount and hypothetic wells in which red and green markers are amplified are exemplified. (E, F) The multiplex reaction products are split into replica plates and a further PCR round carried out with semi-nested primers for each marker (E; shows hypothetical distribution of red marker product and F shows hypothetical distribution of green marker product). (G, H) The PCR products of the semi-nested step are analysed by gel electrophoresis. In this exemplification, the red marker is found in 24 of the wells and the green marker in 46 of the wells, corresponding to a two-fold increase in copy-number.

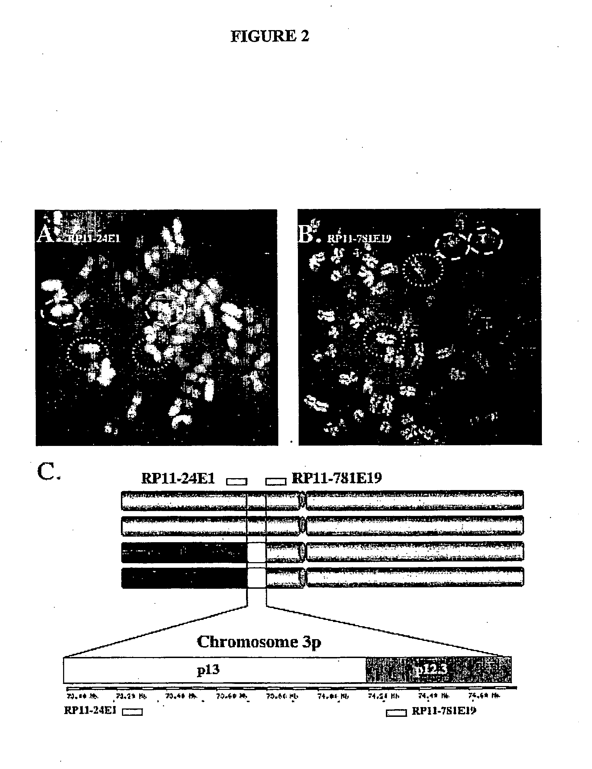

**Figure 2:** In situ *hybridization of BAC clones with SK-RC-9 chromosomes to localise the t(3;5) translocation breakpoint*

The presence of a t(3;5) non-reciprocal chromosomal translocation typical of non-papillary renal cell carcinoma was found in SK-RC-9 cells (an incompletely tetraploid line) using chromosome 3 and 5 painting (Supplementary Fig. 1 online). The regional location of the t(3;5) was determined using FISH with BAC clones from chromosome 3. BAC clones defining an approximately 1Mb region of human chromosome 3 short arm (clone RP11-24E1 located at 73256358-73419679 bp, panel A and clone RP11-781E19, located at 74210885-74236089 bp, panel B) were fluorescently-labelled green for FISH analysis of metaphase spreads from SK-RC-9 chromosomes in combination with whole chromosome 5 paints (red fluorescence). BAC clone RP11-24E1 hybridizes to the short arms of two normal chromosomes 3 (panel A, green-circled), but not to the chimaeric translocated chromosome t(3;5) (panel A, white-circled) indicating that this BAC is telomeric of the translocation t(3;5) breakpoint. BAC clone RP11-781E19 hybridizes to both normal chromosomes 3 (panel B, green-circled) as well as to the chimaeric chromosomes t(3;5) (panel B, white-circled). Thus, this BAC maps centromeric of the t(3;5) breakpoint. These data show that the translocation breakpoint occurs between or within the chromosome region defined by these two BAC clones.
C. Schematic representation of normal chromosomes 3 and chimaeric t(3;5) in SK-RC-9 showing the region of chromosome 3 that contains the putative t(3;5) translocation breakpoint as determined by FISH. Chromosomes 3 are depicted in green and chromosomes 5 in red. The zone containing the breakpoint is represented in white and expanded below, comprising approximately the region of chromosome 3p13-p12.3.

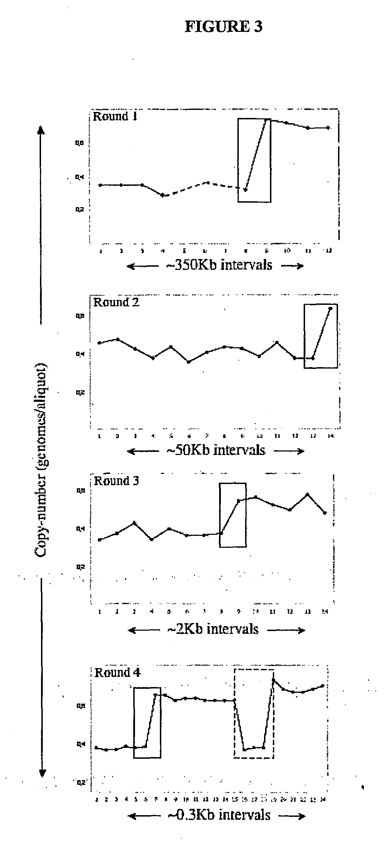

**Figure 3:** *The MCC method localises the t(3;5) break to within 300bp on chromosome 3*

Each graph shows the relative copy-number (vertical axis) of sequences spanning chromosome 3p (horizontal axis; telomeric-centromeric orientation is left to right).
Round 1: Sequences were selected at intervals of about 200 to 500kb spanning ~3.7Mb, encompassed by 3p13-p12.3 (exact chromosomal location and distances together with primer sequences are given in Table 1). Primer sets, namely sets 5 and 7 failed to yield product (indicated by dotted lines). Visual inspection indicated a two fold shift in copy-number between markers 8 and 9 (shown by the open box). In subsequent rounds 2, 3 and 4, new sequences were selected with progressively shorter distances between the markers. Round 4 used markers ~300bp (50-300bp) apart and showed a copy-number shift that represents the t(3;5) non-reciprocal translocation breakpoint (indicated by the open box) and a second abnormality that represents a short deletion of about 700 bp in the region of chromosome 3, (indicated by the dotted box) centromeric of the translocation junction.

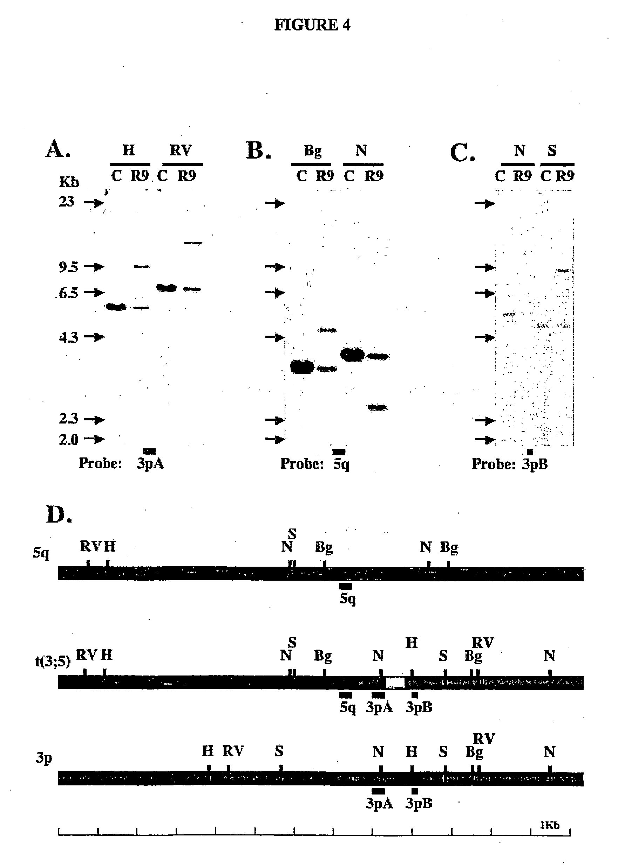

**Figure 4:** *Filter hybridization of SK-RC-9 DNA shows a rearranged segment and reveals an insertion accompanying a micro-deletion*

A-C. Genomic DNA from SK-RC-9 or SK-RC-12 (a renal carcinoma with a der(3;5) proximal to that in SK-RC-9) was digested with the indicated restriction enzymes, fractionated and transferred to filters for hybridization to cloned PCR probes from either chromosome 3p (3pA or 3pB) or 5q. The chromosome 3p probes were located from the MCC data in Figure 3 and a repeat-free genomic sequence from this region was identified using the human genome sequence database. Similarly a probe from chromosome 5q was determined from the human genome sequence database after cloning and sequencing the t(3;5) junction.

D. Partial restriction maps of relevant regions of chromosomes 5q, t(3;5) and 3p showing the location of the hybridization probes on either side of the t(3;5) breakpoint (there were two probes for chromosome 3p, designated 3pA and 3pB). The length of the insertion, associated with the small deletion, is not certain and is indicated by the small grey shaded region which includes a BglII restriction enzyme site as shown by the filter hybridizations shown in B.

B= BglII; H= HindIII; N= NcoI; RV= EcoRV; S= SpeI

C = control genomic DNA from SK-RC-12; R9 = SK-RC-9 genomic DNA

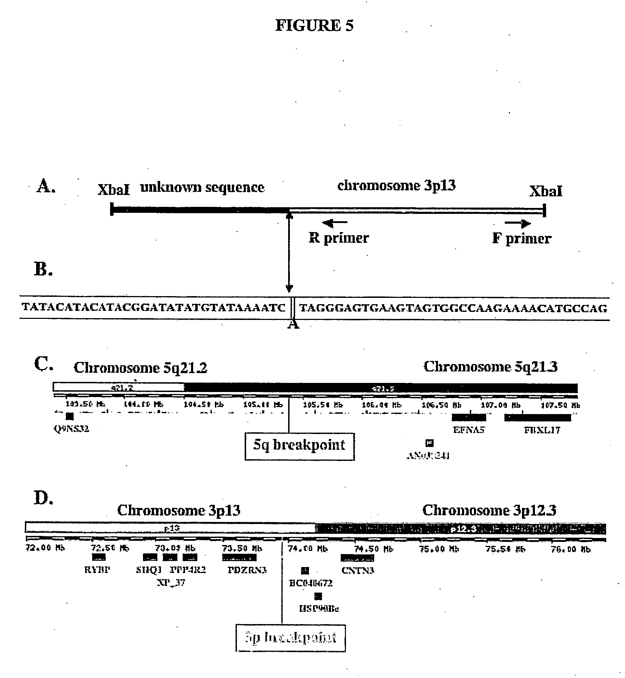

**Figure 5:** *The sequence and chromosomal location of the t(3;5) non-reciprocal translocation junction*

The position of the t(3;5) translocation junction in the SK-RC-9 genome was located to within 300bp by the MCC method. The human genome sequence database allowed identification of restriction sites around this putative translocation and inverse PCR was used to clone the junction. SK-RC-9 DNA was digested with XbaI, self-ligated at high dilution to obtain intra-molecular circles and a PCR product obtained by amplification with the primers F and R (panel A) (see Methods for primer sequences). The sequence of the PCR products confirmed that the junction of the t (3;5) non-reciprocal translocation had been cloned and the junction comprised abutted 5q (panel B, sequence boxed in red) and 3p (panel B, sequence boxed in green) sequences. Note the single additional A residue at the junction of the fused sequence (location shown by arrow) may have derived from either chromosome. The location of the translocation breakpoints were determined in chromosome 5q and 3p using the human Ensembl database (NCBI release 35) and indicated in panels C (5q) and D (3p). In each of these panels, the top line indicates the relevant chromosome bands and Mb distances and below are shown various known or putative genes. The position of Ensembl genes is shown for both chromosome 5 (C) and chromosome 3 (D). A Genscan predicted transcript (AN038241) is shown at 106.58Mb on chromosome 5 and two mRNAs located from on chromosome 3, BC040672 (Riken cDNA) and HSP90 at 74.10 and 74.2 respectively. The t(3;5) translocation of SK-RC-9 does not split genes on either chromosome 3 or chromosome 5.

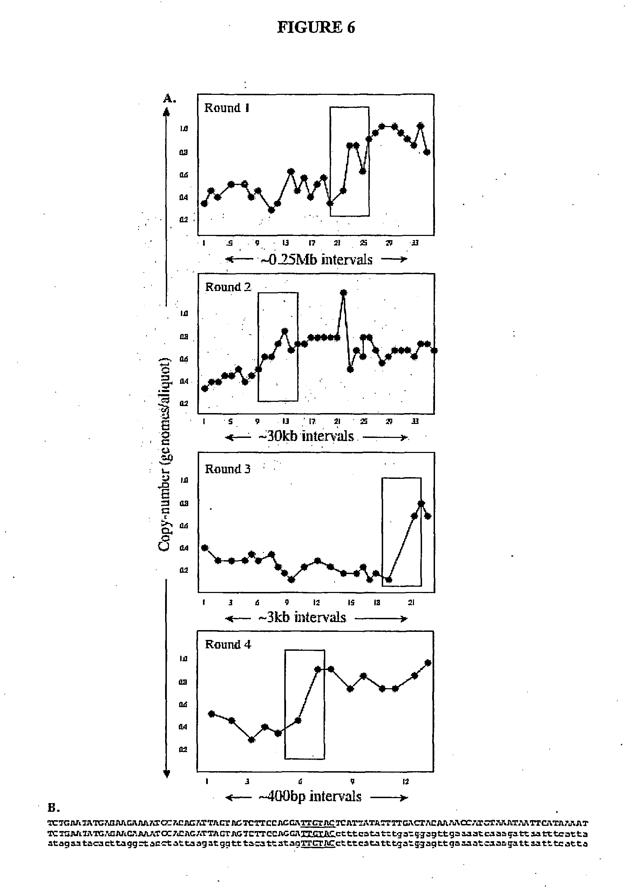

**Figure 6:** *MCC mapping of a chromosome 3 deletion in the SK-RC-12.*

A. For MCC round 1 mapping, a panel of 35 markers were used to screen a genomic region ~9Mb) with marker intervals around 0.25Mb apart. A copy number shift was detected between markers 22 and 23. The results are shown for the marker panel with genomes/aliquot on the y-axis and marker number on the x-axis. Distances between markers do not reflect genomic distances but are numeric ones. The boxed zones represent shifts from low copy and high copy. Based on round 1 data, a second set of primers were designed spaced at an average of 30kb apart, demonstrating that the copy number variation mapped to between markers 9 and 13. Subsequently, two more rounds of MCC was conducted using markers at about 3Kb (round 3) and 400bp apart (round 4). A copy number shift is occurred between markers 6 and 7 in round 4 defining a location of the shift within 800bp of chromosome 3.

B. The genomic region of SK-RC-12 DNA with this copy number shift was cloned after inverse PCR and revealed a cryptic deletion of chromosome 3 in SK-RC-12 cells. The upper sequence (upper case) spans the telomeric end of the chromosome 3p deletion and the lower sequence (lower case) spans the centromeric end of the chromosome 3p deletion while the middle sequence is the fusion found at the junction of the deletion in SK-RC-12 DNA, located at 81.64 and 81.94 Mb.

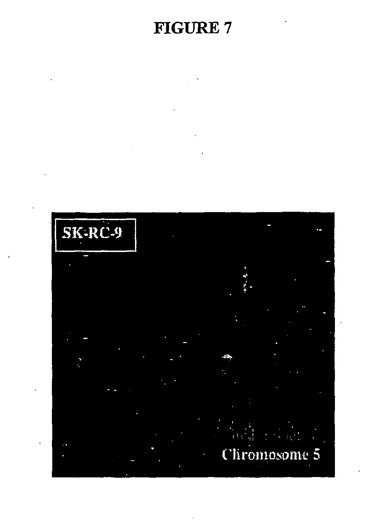

**Figure 7:** *Painting ofSK-R C-9 metaphase chromosomes*

Fused images are shown in hybridisation signals with chromosome 3 (red) or 5 (green) and DAPI staining of metaphase from SK-RC-9 cultures (this line is incompletely tetraploid), showing the presence of non-reciprocal t(3;5) chromosomal translocation typical of non- papillary renal cell carcinoma. Chromosome paints were obtained from Vysis.

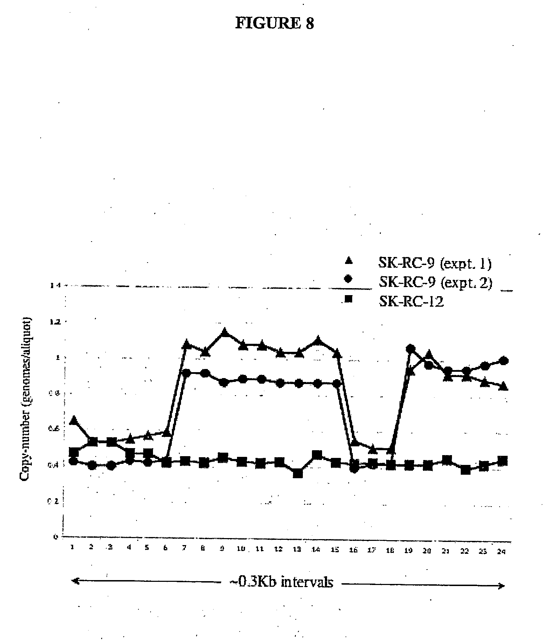

**Figure 8:** *Identification of micro-deletion in SK-RC-9 chromosome t(3;5)*

MCC data were obtained from round 4 PCR (as in main text Fig. 3) using either SK-RC-9 in duplicate experiments or SK-RC-12 as a control. The translocation and micro-deletion regions show reproducible copy-number shifts, whereas the control SK-RC-12 DNA displays a horizontal curve with no copy-number shift in this region.

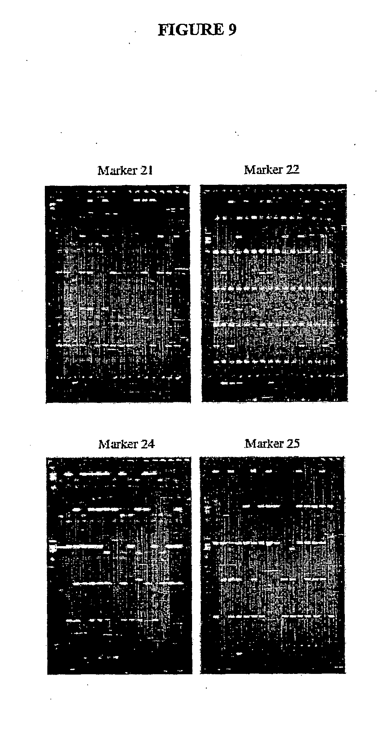

**Figure 9:** *Agarose gel fractionation of PCR products for round one MCC of SK-RC-12 DNA*

Round one MCC analysis was performed with SK-RC-12 DNA using markers spanning a region spanning about 0.25Mb of chromosome 3p (see Fig. 6A, round 1) showing a copy number shift between markers 22 and 23. The analytical gels for seminested PCR products of markers 21, 22, 24 and 25 are shown.

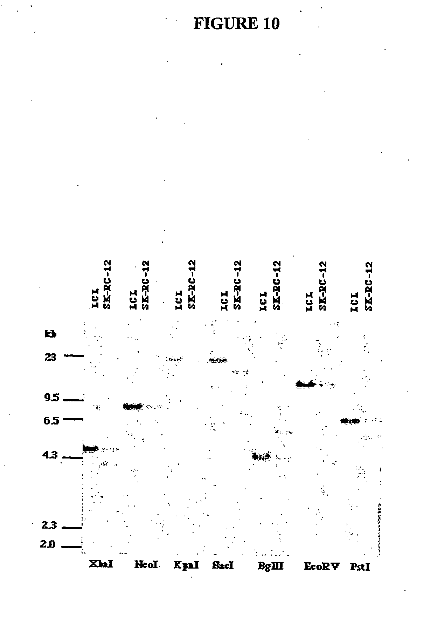

**Figure 10:** *Filter hybridisation of SK-RC-12 DNA to confirm genomic alteration detected by MCC*

MCC of SK-RC-12 chromosome 3p DNA identified a copy number shift to within a 400bp region (see Fig 6A, round 4) indicative of a genomic alteration. This was confirmed by filter hybridisation of SK-RC-12 genomic DNA compared to DNA prepared from a control lymphoblastoid cell line (LCL). A 237bp probe was amplified from chromosome 3 and hybridised to the restriction digests indicated. A rearranged fragment was observed in each case with SK-RC-12 DNA compared to the LCL.

DETAILED DESCRIPTION OF THE INVENTION

ALTERATION

As used herein, the term "alteration" refers to a change - such as a known or a hidden change - that involves the loss or gain of genetic material.

Suitably, the alteration is a copy-number alteration - such as a copy number alteration from the diploid state - which can be determined by counting the DNA reiteration frequency using the methods described herein.

In one embodiment, the alteration is an aberration - such as a chromosomal aberration.

In one embodiment, the alteration is selected from the group consisting of a translocation (eg. an unbalanced translocation or a non-reciprocal translocation), an amplification; a duplication, or a deletion.

In a particularly preferred embodiment, the alteration is a non-reciprocal translocation - such as a non-reciprocal translocation that is detected in nucleic acid from or derived from a cancer or tumour cell.

COPY NUMBER

[0068]   The term "copy number" means the number of copies of a particular nucleic acid sequence (eg. locus) in the genome of a particular organism - such as a human.

TEST MARKER

As used herein, the term "test marker" refers to a nucleic acid sequence, the copy number of which is to be determined according to the methods of the present invention.

REFERENCE MARKER

As used herein, the term "reference marker" refers to a nucleic acid sequence, the copy number of which is already known or is determined in order to calculate the copy number of the test marker. As described herein, the copy number of the test marker is calculated by comparing the number of amplified products for the test marker with those for the reference marker.

The copy number of the reference marker may be determined using the methods described herein or any other method that can be used to determine the copy number of a nucleic acid - such as comparative genome hybridisation or array comparative genome hybridisation and the like. Of course, the copy number of the reference marker may even be determined by reference to the literature since its copy number may have been previously described.

In one embodiment of the present invention, the test marker and/or the reference marker are amplified.

SAMPLE

The term "sample" as used herein refers to a sample comprising or consisting of nucleic acid typically DNA (preferably genomic DNA) - in a form suitable for detection by amplification as described herein. The samples used in the present methods can come from essentially any organism - such as any eukaryotic organism, including, but not limited to, humans, mice, rats, hamsters, horses and cows.

Preferably, the sample is or is derived from a human.

The sample may be derived from essentially any source associated with an organism such as a sample of tissue and/or fluid obtained from an individual or a group of individuals. Illustrative examples include skin, plasma, serum, blood, urine, tears, organs, spinal fluid, lymph fluid and tumours. The sample may even be derived from *in vitro* cell cultures, including the growth medium, recombinant cells and cell components.

When samples are taken for diagnosis or study of a tumour, the sample typically is taken from tumour tissue or tissue suspected of having the beginnings of tumour formation. With enrichment techniques, the necessary sample for assessment of tumour presence can be obtained by enriching tumour cells or DNA from plasma, for example.

Thus, in many instances, the sample will be a tissue or cell sample in which the nucleic acid is be isolated and/or cloned and/or amplified. It may be, e.g., genomic DNA from a particular chromosome, or selected sequences (e.g. particular promoters, genes, amplification or restriction fragments) within particular alterations - such as amplicons or deletions.

Methods of isolating cell and tissue samples are well known to those of skill in the art and include, but are not limited to, aspirations, tissue sections, needle biopsies, and the like. Frequently the sample will be a "clinical sample" which is a sample derived from a patient, including sections of tissues such as frozen sections or paraffin sections taken for histological purposes. The sample can also be derived from supernatants (of cells) or the cells themselves from cell cultures, cells from tissue culture and other media in which it may be desirable to detect chromosomal abnormalities and/or determine copy number.

Standard procedures known in the art can be used to isolate the required nucleic acid from the sample.

A particular application of the methods described herein is for analysing DNA sequences from subject cell(s) or cell population(s), for example, from clinical specimens including tumour and foetal tissues.

However, if the nucleic acid, for example, DNA, is to be extracted from a low number of cells (eg. from a particular tumour subregion) or from a single cell, it may sometimes be necessary to amplify that nucleic acid, by a polymerase chain reaction (PCR) procedure or by a non-polymerase chain reaction (non-PCR) procedure. PCR and preferred PCR procedures are described herein. Exemplary non-PCR procedures include the ligase chain reaction (LCR) and linear amplification by use of appropriate primers and their extension (random priming) as described herein.

Advantageously, nucleic acids from archived tissue specimens, for example, paraffin-embedded or formalin-fixed pathology specimens, may be tested by the methods described herein. The nucleic acid from such specimens may be extracted by known techniques such as those described in Anatomic Pathology 95(2): 117-124 (1991) and Cancer Res. 46: 2964-2969 (1986), and if necessary, amplified for testing. Such nucleic acid can be amplified by using a polymerase chain reaction (PCR) procedure, in which for example, DNA from paraffin-embedded tissues is amplified by PCR.

A particular value of testing such archived nucleic acid is that such specimens are usually associated with the medical records of patients from whom the specimens were taken. Therefore, valuable diagnostic/prognostic associations can be made between the revealed state of patient's nucleic acid material and the medical histories of treatment and outcome for those patients. For example, information gathered by the methods described herein may be used to predict the invasiveness of a tumour based upon its amplification and/or deletion pattern matched to associations made with similar patterns of patients whose outcomes are known.

Analogously, other nucleic acid that is fixed by other methods - such as archaeological material preserved through natural fixation processes - may also be studied. Copy number differences between species provides information on the degree of similarity and divergence of the species studied. Evolutionarily important linkages and disjunctions between and among species that are either extant or extinct can therefore be made by using the methods described herein.

As described herein, once the sample has been prepared, it may be divided into one or more aliquots (eg. a plurality of aliquots). In the context of the number of aliquots, the term "plurality" means 2 or more, preferably 10 or more, preferably 20 or more, preferably 30 or more, preferably 40 or more, preferably 60 or more, preferably 80 or more, preferably 90 or more, or even preferably, 100, 200, 300, 400, 600, 800 or even 1000 or more.

In a particularly preferred embodiment, the methods of the present invention are conducted in one or more plates containing multiple wells. Preferably, the plate contains 96 wells or 384 wells. Plates of other suitable sizes may also be used. Accordingly, the number of aliquots of samples will typically correlate with the number of wells in the plate. However, when such plates are used, the sample is not necessarily aliquoted into each and every well since one or more of the wells may be used as controls. By way of example, if an experiment is conducted in one or more 96 well plates, then about 8 or so of the wells of each plate may be used as negative controls, which are not contacted with the aliquots of the sample. In other words, the negative controls lack the nucleic acid - such as genomic DNA - that is or is derived from the sample.

Each aliquot of the sample used in accordance with the present invention comprises less than one genome per amplification reaction as described for HAPPY mapping (22). Preferably each aliquot of the sample used in accordance with the present invention comprises about 0.9, about 0.8, about 0.7, about 0.6, about 0.5, about 0.4, about 0.3, about 0.2 or about 0.1 genomes or less per amplification reaction. More preferably, each aliquot of the sample used in accordance with the present invention comprises a range consisting of any suitable start or end point of the number of genomes per amplification reaction- such as about 0.1-0.9 genomes per amplification reaction or about 0.3-0.5 genomes per amplification reaction. Most preferably, each aliquot of the sample used in accordance with the present invention comprises about 0.3 genomes per amplification reaction.

To determine the precise DNA concentration in the sample prior to aliquoting, initial tests may be performed using a range of DNA dilutions which are expected (based on the DNA concentration determined by UV spectrophotometry) to give between 0.25 and 8 genomes of DNA per sample. Around 4 nucleic acid sequences per nucleic acid sample can be analysed at different dilutions - such as 6 dilutions - for different nucleic sequences that are believed to be present at only one copy per haploid genome. The proportion of samples at each dilution found positive for these markers is used to refine the estimate of the nucleic acid concentration and hence determine the dilution required for the subsequent analysis.

AMPLIFICATION

As used herein, "amplification" refers to any process for multiplying strands of nucleic acid - such as genomic DNA - *in vitro.*

Preferably, the process is enzymatic and is linear or exponential in character.

Amplification techniques include, but are not limited to, methods broadly classified as thermal cycling amplification methods and isothermal amplification methods. Suitable thermal cycling methods include, for example, ligase chain reaction (Genomics 4:560, (1989); and Science 241: 1077 (1988)). The ligase chain reaction uses DNA ligase and four oligonucleotides, two per target strand. The oligonucleotides hybridise to adjacent sequences on the nucleic acid to be amplified and are joined by the ligase. The reaction is heat denatured and the cycle repeated. Isothermal amplification methods useful in the present invention include, for example, Strand Displacement Amplification (SDA) (Proc. Nat. Acad. Sci. USA 89:392-396 (1992)), Q-beta-replicase (Bio/Technology 6:1197-1202 (1988)); nucleic acid-based Sequence Amplification (NASBA) (Bio/Technology 13:563-565 (1995)); and Self-Sustained Sequence Replication (Proc. Nat. Acad. Sci. USA 87:1874-1'878 (1990)).

A particularly preferred amplification method is PCR. As is well known to a person skilled in the art, this is a method in which virtually any nucleic acid sequence can be selectively amplified. The method involves using paired sets of oligonucleotides of predetermined sequence that hybridise to opposite strands of DNA and define the limits of the sequence to be amplified. The oligonucleotides prime multiple sequential rounds of DNA synthesis catalysed by a thermostable DNA polymerase. Each round of synthesis is typically separated by a melting and re-annealing step, allowing a given DNA sequence to be amplified several hundred-fold in less than an hour (Science 239:487, 1988). As described herein, more than one nucleic acid can be simultaneously amplified by multiplex PCR in which multiple paired primers are employed.

The nucleic acid may be labelled at one or more nucleotides during or after amplification.

As described herein, the methods are carried out using a two-phase amplification reaction.

*First amplification reaction*

The first amplification reaction is typically an amplification step with multiplexed outer primers in order to amplify one or more, preferably two or more (eg. a plurality) of nucleic acid sequences. When analysing large genes and transcripts or undefined genes, multiple individual amplification reactions are often required to identify alterations. Thus, to streamline the analysis of large complex genes, multiplex primers for the simultaneous amplification of different nucleic acid sequences in a single amplification reaction may be utilised. Methods for the preparation of multiplex primers are known in the art, as reported in, for example, US 6,207,372.

In one embodiment, one or more markers are amplified (eg. a test marker and/or a reference marker).

In another embodiment, two or more markers are amplified (eg. a test marker and/or a reference marker).

In another embodiment, all markers are amplified.

In another embodiment, all copies of one or more sequences or markers are amplified.

In another embodiment, one or more markers, two or more markers, or all markers in a chromosomal or genomic region or locus of interest are amplified.

For the first reaction, nucleic acid is prepared and diluted to less than about one genome per aliquot. If the amplification method of choice is PCR then a master mix may be prepared containing the primers for all sequences to be amplified in PCR buffer - such as Gold PCR buffer (Perkin-Elmer) - MgCl2 - such as about 2mM MgCl2, dNTPs - such as about 200$\mu$M of each dNTP and Taq DNA polymerase - such as about 0.1U/$\mu$l of Taq Gold DNA polymerase (Perkin-Elmer).

Suitably, the nucleic acid is divided into a plurality of aliquots.

Suitably, the nucleic acid is divided into a plurality of identical or substantially identical aliquots.

Suitably, each of the aliquots that is prepared is dispensed into a well or a receptacle.

Accordingly, in one embodiment of the present invention, the amplification reactions will be performed in parallel using, for example, 96 well plates. Thus, aliquots of the master mix will be distributed into, for example, 8 wells of the 96 well plates (negative controls), and subsequently, approximately 0.03 genomes/$\mu$l of the genomic DNA added to the mastermix for analysis. The aliquots of this mix (each containing about 0.3 genomes of DNA) are aliquotted into each of the remaining wells and the control wells receive an equivalent mixture but lacking genomic DNA (negative controls). All samples are overlaid with mineral oil (about 20μl).

An initial amplification reaction may be performed to amplify one or more markers in each of the aliquots. Preferably, all of the markers in each of the aliquots is amplified. Thermocycling is typically carried out with hot start at about 93°C for about 9 minutes, followed by about 25 cycles of about 20 seconds at about 94°C, about 30 seconds at about 52°C and about 1 minute at about 72°C. The skilled person will recognise that variations of this thermocycling reaction may be made.

Each of the amplified products are diluted to an amount that is sufficient for the second amplification reaction to amplify the nucleic acid contained therein.

In one embodiment of the present invention it is preferred that the first amplification reaction is automated.

*Second amplification reaction*

Suitably, one or more (eg. each) of the amplified products from the first amplification reaction are subdivided or split into one or more replica samples or replica aliquots.

Suitably, one or more (eg. each) of the amplified products from the first amplification reaction is subdivided, split or dispensed into one or more replica wells or replica receptacles.

Accordingly, prior to the second amplification reaction one or more replica sets of amplified products from the first amplification reaction is prepared. Suitably, the replica set of amplified products comprises 50%, 60%, 70%, 80%, 90%, 95%, 96%, 97%, 98%, 99% or 100% of each of the amplified products from the first amplification reaction. Preferably, the replica set of amplified products comprises 90%, 95%, 96%, 97%, 98%, 99% or 100% of each of the amplified products from the first amplification reaction.

Typically, one or more (eg. each) aliquot from the first amplification reaction is subdivided or split or dispensed into a number of replica samples, aliquots, wells or receptacles that correlates with the number of markers that are amplified. Suitably, one marker is amplified in each of the replica samples, aliquots, wells or receptacles. Thus, by way of example, if four markers are amplified then each aliquot from the first amplification reaction is subdivided, split or dispensed into four replica samples, aliquots, wells or receptacles. A first marker will be amplified in a first of the replica samples, aliquots, wells or receptacles, a second marker will be amplified in a second of the replica samples, aliquots, wells or receptacles and so on.

In one embodiment of the present invention, one or more of the amplification products obtained or obtainable from the first amplification reaction is amplified.

Typically, the second amplification reaction is performed using a semi-nested amplification reaction in which the same reverse primer as used in the first amplification reaction is used in combination with a forward-internal primer for each marker to be amplified.

If the second amplification is performed using PCR then typically, the semi-nested PCR utilises $MgCl_2$ - such as about 1.5mM $MgCl_2$, and about 1μM of the relevant forward-internal and reverse primers. The other concentrations are about the same as those used for the first amplification reaction as before.

In one embodiment, the thermocyclcing is performed at about 93°C for about 9 minutes, followed by about 33 cycles of about 20 seconds at about 94°C, about 30 seconds at about 52°C and about 1 minute at about 72°C). The skilled person will recognise that variations of this thermocycling reaction may be made.

Advantageously, the second-phase amplification reactions are set up robotically in multiple 384-well microtitre plates such that each of the 96 first amplification reactions can be screened for each of the different markers. Thus, by way of example, if four nucleic acid sequences are screened in the second amplification, then a 384 well plate can be used thereby providing 96 wells for each of the four nucleic acid sequences. Alternatively, the second, semi-nested amplification stage may be carried out in a second 96-well plate if a multi-channel pipette is used for transfers, rather than a robotic system.

In one embodiment of the present invention it is preferred that the second amplification is automated.

In one embodiment of the present invention it is preferred that the first and second amplification reactions are automated.

Advantageously, the methods described herein can be used in combination with other amplification reactions. By way of example, the methods described herein may be used in combination with one or more PCR reactions that detect genetic aberrations - such as reciprocal translocations.

In a further aspect, there is provided a method of measuring the copy number frequency of one or more nucleic acid sequences in a sample, comprising the steps of:

(a) providing a plurality of aliquots of the sample, wherein each aliquot comprises nucleic acid in an amount that is less than one genome per aliquot; (b) amplifying one or more nucleic acid sequences from each of the plurality of aliquots in a first amplification reaction; (c) amplifying in a second amplification reaction one or more nucleic acid sequences from each of the plurality of aliquots prepared according to step
(b) wherein at least one of the nucleic acid sequences is a test marker; and (d) calculating the copy number of the test marker by comparing the number of amplified products for the test marker with those for a reference marker.

In a further aspect, there is provided a method of measuring the copy number frequency of one or more nucleic acid sequences in a sample; comprising the steps of:

(a) providing a plurality of aliquots of the sample, wherein each aliquot comprises nucleic acid in an amount that is less than one genome per aliquot; (b) amplifying one or more nucleic acid sequences from each of the plurality of aliquots in a first amplification reaction; (c) amplifying in a second amplification reaction two or more nucleic acid sequences from each of the plurality of aliquots prepared according to step
(b) wherein at least one of the nucleic acid sequences is a test marker and at least one of the nucleic acid sequences is a reference marker; and (d) calculating the copy number of the test marker by comparing the number of amplified products for the test marker with those for the reference marker.

In a further aspect, there is provided a method of measuring the copy number frequency of one or more nucleic acid sequences in a sample; comprising the steps of:

(a) providing one or more (eg. a plurality of) aliquot(s) of the sample, wherein each aliquot comprises nucleic acid in an amount that is less than one genome per aliquot;
(b) amplifying one or more (preferably, all) markers from each aliquot(s) in a first amplification reaction; (c) dispensing each of the amplification products from the first amplification reaction into replica samples; (d) amplifying in a second amplification reaction one or more markers from each of the aliquot(s) in the replica samples prepared according to step (c), wherein at least one of the nucleic acid sequences is a test marker; and (e) calculating the copy number of the test marker by comparing the number of amplified products for the test marker with those for the reference marker.

In a further aspect, there is provided a method of measuring the copy number frequency of one or more nucleic acid sequences in a sample, comprising the steps of:

(a) providing one or more (eg. a plurality of) aliquot(s) of the sample, wherein each aliquot comprises nucleic acid in an amount that is less than one genome per aliquot;
(b) amplifying one or more (preferably, all) markers from each aliquot(s) in a first amplification reaction; (c) dispensing each of the amplification products from the first amplification reaction into replica samples; (d) amplifying in a second amplification reaction two or more markers from each of the aliquot(s) in the replica samples prepared according to step (c), wherein at least one of the nucleic acid sequences is a test marker and at least one of the nucleic acid sequences is a reference marker; and (e) calculating the copy number of the test marker by comparing the number of amplified products for the test marker with those for the reference marker.

## PRIMERS

The primers used in the amplification are selected so as to be capable of hybridising to sequences at flanking regions of the nucleic acid sequence (eg. locus) being amplified. The primers are chosen to have at least substantial complementarity with the different strands of the nucleic acid being amplified.

The primer must have sufficient length so that it is capable of priming the synthesis of extension products in the presence of an agent for polymerisation. The length and composition of the primer depends on many parameters, including, for example, the temperature at which the annealing reaction is conducted, concentration of primer and the particular nucleic acid composition of the primer. Typically the primer includes 15-30 nucleotides, preferably, 18-20bp. For some embodiments of the present invention, it is preferred that the primers have a Tm between about 52-60°C (based on the calculation $Tm=2x(A+T)+4x(G+C)$). Preferably, the design includes at least two G or C bases at the 3' end and at least one at the 5' end. Typically, primers that have runs of any single base longer than 4 bases are not used. Internal amplimer length is designed to be between about 80-150bp and the position of the external primer no more than about 150bp upstream of forward-internal primer.

The length of the primer may be more or less depending on the complexity of the primer-binding site and the factors listed above in addition to those that are known to the skilled person.

Typically, the primers hybridise specifically to a particular nucleotide sequence. The term "hybridise specifically" as used herein refers to the binding, duplexing, or hybridizing of a nucleic acid preferentially to a particular nucleotide sequence under stringent conditions. The term "stringent conditions" refers to conditions under which a primer will hybridise preferentially to its target subsequence, and to a lesser extent to, or not at all to, other sequences.

Primers can be synthesised according to the methods that are well known in the art.

Primer selection may be conducted using various methods that are known in the art (eg. similar to design for conventional uses of PCR) including, for example, Universal Primer Designer after masking repetitive elements from the genomic sequence (Assembly NCBI 35, http://www.ensembl.org) using Repeatmasker (http://www.repeatmasker.org).

In the first amplification reaction as described above, primers are typically used that comprise a forward and a reverse primer for each nucleic acid sequence that is amplified. The multiple primer pairs may be combined in a single multiplex reaction such that in the first amplification reaction, multiple nucleic acid sequences - such as all markers - are amplified.

In the second amplification reaction as also described above, a semi-nested amplification reactions is typically performed for each specific nucleic acid sequence that is to be amplified. The primers used are suitably a reverse primer as used in the first amplification reaction in combination with a forward-internal primer.For some embodiment of the present invention it is preferred that the primers used are forward and reverse-internal primers. For some embodiment of the present invention it is preferred that the primers used are the same or substantially the same as those used in the first amplification reaction. Typically about 0.15 $\mu$M of each primer will be used in first amplification reaction. Typically about 1$\mu$M of the relevant forward-internal and reverse primers will be used for the second amplification reaction.

In one embodiment of the present invention, several primer pairs each for a particular chromosomal rearrangement in a single reaction are used. Each pair of primers results in an amplification product that is of a different length and so the amplification products can be differentiated.

In a further embodiment of the present invention, labeled primers are used in which each primer pair is labeled with a different label and so the amplification products can be differentiated.

Advantageously, multiple chromosomal rearrangements may therefore be analysed.

DETERMINING COPY NUMBER

**[0135]** After the second amplification reaction the amplification products may be analysed in various ways to determine the copy number of the amplified sequences.

By way of example, the amplification products may be analysed by separating the amplified products using electrophoresis in order to score the presence or absence of amplification product(s) in each aliquot of the sample.

By way of further example, the results may be scored by melting-curve analysis.

The results may be assessed by visual assessment of the number of positive wells. By way of example, if two nucleic acid markers (A and B) are scored on the same set of 88 aliquots, and if the numbers of aliquots scoring positive for each marker were 34 and 56 respectively, then the average concentrations of the two sequences can be calculated as 0.49 and 1.0 copies per aliquot, respectively. Hence, if sequence A is known to be present at N copies per genome, it can be inferred that sequence B is present at 2N copies per genome.

If nucleic acid molecules are distributed randomly amongst N aliquots to give an average of Z molecules per aliquot then, according to the Poisson distribution, the number of aliquots Np which are expected to contain at least one molecule of the nucleic acid is given by the equation:

$$Np = N(1 - e^{-z}) \qquad (i)$$

Conversely, if a panel of N sub-genomic aliquots of nucleic acid is tested for the presence of a given sequence, and if Np of these aliquots score positive for the sequence, (i.e. contains at least one copy of the sequence) then the average number of molecules of that sequence per aliquot can be calculated as:

$$Z = -\ln(1 - Np/N) \qquad (ii)$$

Hence, from the number of aliquots scoring positive for any given sequence, the concentration of that sequence (expressed in copies per aliquot) may be determined. If two or more sequences are analysed in this way on the same set (or similar sets) of aliquots of nucleic acid, then their relative concentrations and hence their relative abundance can be calculated.

IDENTIFYING ALTERATIONS

In a further aspect of the present invention there is provided a method of identifying one or more alterations in a sample of nucleic acid, comprising the steps of: (a) determining the copy number of one or more nucleic acid sequences in a sample of nucleic acid according to the method of the first aspect of the present invention; and (b) iteratively repeating the method at progressively higher resolutions.

Accordingly, the method of the first aspect of the present invention is iteratively repeated by amplifying nucleic acid sequences that are spaced at progressively smaller intervals. Typically, a crude picture of a genomic alteration may be obtained using methods already known in the art - such as fluorescent *in situ* hybridisation (FISH). Such methods may be useful for providing an estimate of the copy number of sequences on a particular chromosome in the vicinity of a genomic alteration - such as a breakpoint, for example. However, in order to further define the genomic alteration of interest the method of the present invention can advantageously be used by iteratively repeating it at progressively higher resolutions. Thus, for example, in a first round, markers spaced at intervals of about 0.2-0.5Mb over about 3.8Mb in the region of chromosome in which the genomic alteration has previously been found to occur may be used. A subsequent round may be performed using markers at intervals of about 50kb in order to further refine the putative genomic alteration to within about 40kb. Still further rounds may be performed to further localise the genomic alteration to approximately 1-4kb and then to less than 500bp. At such fine resolution, the methods may even be used to identify other genomic alterations - such as deletions on the centromeric side of a translocation.

DISEASE

Many chromosomal regions or specific genes have been identified as being present at altered copy number in diseased cells. The copy number for any particular nucleic acid sequence is typically 2 in a diploid individual, reflecting the presence of one copy of a nucleic acid sequence on each chromosome. It is widely accepted that copy number changes cause abnormal levels, or activity, of proteins encoded by these regions and ultimately the eventual disease phenotype.

In one aspect, there is provided a method of identifying one or more alterations in a sample of nucleic acid, comprising the steps of: (a) measuring the copy number frequency of one or more nucleic acid sequences in a first sample and a second sample according to the method of the first aspect of the present invention; (b) iteratively repeating the method at progressively higher resolutions for each of the samples; and (c) identifying one or more differences in the copy number frequency of one or more nucleic acid sequences in the first and second samples.

In a preferred embodiment the samples are or are derived from diseased and non-diseased subjects. In a particularly preferred embodiment, the diseased nucleic acid is or is derived from a subject that is suffering from cancer. Accordingly, the method can be used for identifying one or more alterations between a cancer genome and a normal genome.

The invention also provides a method of diagnosing a disease in a subject that is suffering from or is suspected to be suffering from the disease based upon detecting changes in the copy number of a nucleic acid. The subject being tested may exhibit symptoms of the disease or symptoms that can be associated with the disease. The subject may not exhibit any symptoms at all but may instead be suspected of being susceptible or pre-disposed to the disease through family history or genetic testing (eg. genetic fingerprinting), for example, as described herein below. The subject may be a subject that has been exposed to one or more agents or conditions that are known or are suspected to cause the disease. The subject may even be a subject that is in the process of or is suspected to be in the process of developing a disease.In one aspect there is provided a method of diagnosing a disease in a subject that is suffering from or is suspected to be suffering from the disease, comprising the steps of: (a) measuring the copy number frequency of one or more nucleic acid sequences in a sample according to the method of the first aspect of the present invention; and (b) comparing the copy number of the one or more nucleic acid sequences with the normal (eg. non-diseased) copy number of the one or more nucleic acid sequences; wherein a difference between the copy number is indicative that the subject is suffering from the disease.

Typically, the normal copy number will be determined by analysing samples from a subject or a plurality of subjects that do not suffer from the disease that is being tested for.

A difference between the copy number of the one or more nucleic acid sequences in the subject being tested and the normal copy number is indicative that an alteration is present and that the subject is suffering from the disease.

A sample from a number of different individuals known to have a common disease may even be used. Screening tests to determine the copy number frequency at a number of different nucleic acid sequences for each of the diseased individuals to identify those nucleic acid sequences having altered copy number may be performed. For example, for individuals having a tumour, a sample may be taken from the tumorous region for each individual and screens performed to identify regions of the genome having altered copy number. With such information, correlations between nucleic acid sequences having altered copy number and particular diseases can be made. Hence, the methods of the present can be used to identify the alteration of nucleic acid sequences, which are associated with specific diseases.

Thus, in a further aspect there is provided a method of diagnosing a disease in a subject that is suffering from or is suspected to be suffering from the disease is known to be pre-disposed to the disease (eg. through family history or genetic testing - such as genetic fingerprinting), has been exposed to one or more agents or conditions that are known or are suspected to cause the disease or is in the process of or is suspected to be in the process of developing the disease comprising the steps of: (a) measuring the copy number frequency of one or more nucleic acid sequences in a sample that are known to be the cause of or associated with the disease; and (b) comparing the copy number of the one or more nucleic acid sequences in the sample with the copy number frequency of the one or more nucleic acid sequences in the sample that is known to be the cause of or associated with the disease; wherein a correlation between the copy numbers is indicative that the subject is suffering from the disease.

In one embodiment, the disease may be cancer - such as kidney cancer.

This invention further provides for a method to detect one or more alterations - such as amplifications and/or deletions - in one or more samples - such as samples that are or are derived from tumour cells. The results that are obtained may be used to determine the subsequent behaviour of the sample. The determination may be made by associating the patterns of alterations in the sample with the behaviour of that sample. Such associations may be made by testing, for example, DNA from archived samples linked to medical records, or when fresh samples are tested by the methods described herein and the patients followed.

Another aspect of the present invention is to provide a method of analysing cells from a suspected abnormal cell or tissue, preferably, at an early stage of development. An advantage of the methods described herein is that only a small amount of genomic nucleic acid is required for the analysis. The early detection of alterations - such as amplifications and/or deletions - in such cells or tissues allows for early therapeutic intervention that can be tailored to the genetic rearrangements. Moreover, such early detection provides a means to associate the progression of the cells or tissues with the genetic rearrangements detected by the methods of this invention.

Using these methods screens can rapidly and inexpensively be performed. Hence, the methods may be well-suited to the development of molecular pathology profiles which allow doctors to make more informed patient prognoses and to better predict patient response to different therapies, thus improving clinical outcomes.

For patients having symptoms of a disease, the method described herein may also be used to determine if the patient has copy number alterations which are known to be linked with diseases that are associated with the symptoms the patient has. For example, for a patient having a tumour, a doctor would obtain a sample of the tumour. Screening of the tumour sample to identify whether there is a copy number alteration at nucleic acid sequences known to be associated with the particular tumour type can rapidly be accomplished using the methods described herein. With specific information regarding copy number alterations and knowledge of correlations between disease outcomes and the effectiveness of different treatment strategies for the particular alteration(s), the doctor can make an informed decision regarding patient prognosis and the most effective treatment option. For example, if the methods show that a particular nucleic acid sequences is amplified and that amplification of that locus is associated with poor recovery, the doctor can counsel the client regarding the likely effectiveness of aggressive treatment options and the option of simply foregoing such treatments, especially if the disease is quite advanced. On the other hand, if the copy number is altered at a locus which is associated with good recovery, the doctor can describe a range of treatment options varying from simply monitoring the disease to see if the condition worsens or more aggressive measures, to ensure that the disease is attacked before it gets worse.

Thus, in a further aspect, there is provided a method for determining if a subject has one or more copy number alterations which are known to be linked with a disease, comprising the step of identifying whether there is a copy number alteration using the method according to the first aspect of the present invention at one or more nucleic acid sequences that are known to be associated with the disease that the subject is suspected to be suffering from, wherein a correlation between the copy number alteration in the subject and the copy number alteration in the disease is indicative that the subject is suffering from the disease.

CANCER

The terms "tumour" or "cancer" refer to the presence of cells possessing characteristics typical of cancer-causing cells - such as uncontrolled proliferation, immortality, metastatic potential, rapid growth and proliferation rate, and certain characteristic morphological features.

Often, cancer cells will be in the form of a tumour, but such cells may exist alone within an animal, or may be a non-tumorigenic cancer cell - such as a leukemia cell.

Cancers include, but are not limited to melanomas, breast cancer, lung cancer, bronchus cancer, colorectal cancer, prostate cancer, pancreas cancer, stomach cancer, ovarian cancer, urinary bladder cancer, brain or central nervous system cancer, peripheral nervous system cancer, esophageal cancer, cervical cancer, uterine or endometrial cancer, cancer of the oral cavity or pharynx, liver cancer, kidney cancer, testis cancer, biliary tract cancer, small bowel or appendix cancer, salivary gland cancer, thyroid gland cancer, adrenal gland cancer, osteosarcoma, chondrosarcoma, and the like.

Tumours can be karyotypically heterogeneous containing various populations of cells each having different types of genetic rearrangements. Tumour cells are difficult to culture, and it is not clear that cultured cells are representative of the original tumour cell population. The present invention provides the means to by-pass the culturing obstacle since only low amounts of nucleic acid are required and therefore allows genetic characterisation of tumour cells and thus, of the heterogeneity of tumours.

Bulk extraction of the nucleic acid from many cells of a tumour can also be used to test for consistent alterations within a tumour.

In a further aspect of the present invention, there is also provided a method for detecting, diagnosing or determining the susceptibility of a subject to cancer comprising the step of determining the presence of a non-reciprocal t(3;5) translocation as described herein in a subject, wherein the presence of the translocation is indicative that the subject has, is likely or is predisposed to developing cancer. Preferably, the cancer is renal cell carcinoma.

## FURTHER APPLICATIONS

Advantageously, the methods described herein provide a rapid, accurate and inexpensive way to determine the copy number frequency of one or more nucleic acid sequences.. This makes the methods ideal for the molecular analyses of numerous diseases, as well as assessment of chromosomal imbalances associated with, for example, health threatening syndromes.

*Prognosis*

The methods described herein may be used to develop correlations between certain disease phenotypes and patient prognosis. For example, the methods can be used to screen numerous nucleic acids from a variety of different patients having the same apparent disease symptoms to identify those nucleic acids which have an abnormal copy number. In this case, samples would be obtained from the diseased tissue. A health history for each test individual can be maintained to make a correlation between nucleic acids having altered copy number and disease outcomes. In this way, correlations between copy number changes and patient prognosis can be made.

Accordingly, in a further aspect there is also provided a method for determining a patient's prognosis comprising the steps of: (a) determining the copy number frequency of one or more nucleic acids from one or more subjects having the same apparent disease symptoms as the patient using the method according to the first aspect of the present invention; (b) identifying those nucleic acids which have an abnormal copy number; (c) correlating the nucleic acids having altered copy number with the disease outcome; and (d) predicting the disease outcome in the patient.

*Optimal Treatment Strategies*

The methods described herein can be routinely applied before administering a drug to a patient for the first time. If the patient is found to lack both copies of a gene expressing an enzyme required for detoxification of a particular drug, the patient generally should not be administered the drug or, should be administered the drug in smaller doses compared with patients having normal levels of the enzyme. The latter course may be necessary if no alternative treatment is available. If the patient is found to lack both copies of a gene expressing an enzyme required for activation of a particular drug, the drug will have no beneficial effect on the patient and should not be administered. Patients having one wild-type copy of a gene and one mutant copy of a gene, and who are at risk of having lower levels of an enzyme, should be administered drugs metabolised by that enzyme only with some caution, again depending on whether alternatives are available. If the drug is detoxified by the enzyme in question, the patient should in general be administered a lower dose of the drug. If the drug is activated by the enzyme, the heterozygous patient should be administered a higher dosage of the drug. The reverse applies for patients having additional copies of a particular biotransformation gene, who are at risk of having elevated levels of an enzyme. The more rational selection of therapeutic agents that can be made with the benefit of screening results in fewer side effects and greater drug efficacy in poor metabolising patients.

The methods may also be useful for screening populations of patients who are to be used in a clinical trial of a new drug. The screening identifies a pool of patients, each of whom has wild-type levels of the full complement of biotransformation enzymes. The pool of patients are then used for determining safety and efficacy of the drugs. Drugs shown to be effective by such trials are formulated for therapeutic use with a pharmaceutical carrier such as sterile distilled water, physiological saline, Ringer's solution, dextrose solution, and Hank's solution.

*Susceptibility to Disease*

The methods described herein may also be used to screen individuals that know they are susceptible to a disease. In this scenario, for example, the individual would know from test results or family history showing the presence of a disease marker that he or she was susceptible to a particular disease. A sample would be removed from the tissue, which the disease to which a patient is susceptible is associated. By way of example only, if the patient comes from a family with a history of skin cancer, a doctor would perform a biopsy of the skin to obtain the sample. A copy number frequency value of the locus or loci associated with the particular disease to which the patient is susceptible can then be determined using the methods described herein. If the determination shows an abnormal copy number, the patient can then be counselled regarding the likelihood that the patient will begin suffering from disease and the pros and cons regarding different treatment alternatives. In this instance in which the patient is not yet exhibiting symptoms of disease, the most appropriate action may be simply to closely monitor the patient. However, the patient, after appropriate counselling, may chose to take aggressive pre-emptive action to avoid problems at a later date.

*No Symptoms of Disease and not Known to be Susceptible to Disease*

The methods described herein can also be useful in screening individuals, which have no symptoms of disease or no known susceptibilities, to disease. An individual in this category would generally have no disease symptoms, have no family history of disease and have no knowledge that he or she carried a marker associated with a disease. In such cases, the methods can be used as a preventive screening tool. In this regard, a number of selected loci known to be associated with certain diseases can be examined to identify loci with aberrant copy number. In this case, samples would be obtained from different tissues or fluids that are affected by the disease(s) being tested for. If a locus or loci were identified that had an altered copy number, then the patient would be advised regarding the likelihood that the disease would manifest itself and the range of treatment options available.

*Prenatal Diagnostics*

Another use of the methods described is in the area of prenatal diagnostics, in particular, as a way to identify copy number abnormalities in an embryo or foetus. An increasingly common trend is for women to wait until later in life to have children. Associated with this delay, is an increased risk that the child will be born with a congenital birth defect.
Methods of obtaining nucleic acid from embryonic (i.e., the developing baby from conception to 8 weeks of development) or foetal (i.e. the developing baby from ninth weeks of development to birth) cells are well known in the art. Examples include but are not limited to maternal biopsy (e.g., cervical sampling, amniocentesis sampling, blood sampling), foetal biopsy (e.g., hepatic biopsy) and chorionic vilus sampling (US 6,331,395).
Isolation of foetal nucleic acid from maternal blood is preferably used according to this aspect of the present invention since it is a non-invasive procedure which does not pose any risk to the developing baby (Am. J. Hum. Genet. (1998) 62(4): 768-75). Cell free foetal nucleic acid can be collected from maternal circulation and analysed as described above (Am. J. Obstet. Gynecol. (2002) 186:117-20). PCR techniques are typically used in conjunction with these methods in order to increase the relative amount of foetal nucleic acid and thus permit analysis.

<u>NUCLEIC ACID</u>

The term "nucleic acid" as used herein refers to a deoxyribonucleotide or ribonucleotide in either single- or double-stranded form.
The term encompasses nucleic acids, *i.e.*, oligonucleotides, containing known analogues of natural nucleotides which have similar or improved binding properties.
The term also encompasses nucleic-acid-like structures with synthetic backbones. DNA backbone analogues include phosphodiester, phosphorothioate, phosphorodithioate, methylphosphonate, phosphoramidate, alkyl phosphotriester, sulfamate, 3'-thioacetal, methylene(methylimino), 3'-N-carbamate, morpholino carbamate, and peptide nucleic acids (PNAs); see Oligonucleotides and Analogues, a Practical Approach, edited by F. Eckstein, IRL Press at Oxford University Press (1991); Antisense Strategies, Annals of the New York Academy of Sciences, Volume 600, Eds. Baserga and Denhardt (NYAS 1992); Milligan (1993) J. Med. Chem. 36:1923-1937; Antisense Research and Applications (1993, CRC Press). PNAs contain non-ionic backbones, such as N-(2-aminoethyl)glycine units. Phosphorothioate linkages are described in WO 97/03211; WO 96/39154; Mata (1997) Toxicol. Appl. Pharmacol. 144:189-197. Other synthetic backbones encompasses by the term include methylphosphonate linkages or alternating methylphosphonate and phosphodiester linkages (Strauss-Soukup (1997) Biochemistry 36: 8692-8698), and benzylphosphonate linkages (Samstag (1996) Antisense Nucleic Acid Drug Dev 6: 153-156).
The nucleic acid may be DNA or RNA of genomic, synthetic or recombinant origin e.g. cDNA. The nucleic acid may be double-stranded or single-stranded whether representing the sense or antisense strand or combinations thereof.

Preferably, the nucleic is DNA, more preferably genomic DNA.

The nucleic acid may be prepared by use of recombinant DNA techniques (e.g. recombinant DNA).

In one aspects of the present invention there is provided an isolated nucleic acid encoding a non-reciprocal t (3;5) translocation, wherein chromosome 5q21.3 to co-ordinate 105386443bp is fused to chromosome 3p13 from co-ordinate 74111893bp and wherein the adenine residue at the junction is from either chromosome.

Preferably, chromosome 5q21.3 comprises the sequence TATACATACATACGGATATATGTATAAAATC or a variant, homologue deviate or fragment thereof.

Preferably, chromosome 3p13 comprises the sequence TAGGGAGTGAAGTAGTGGCCAAGAAAACATGCCAG or a variant, homologue deviate or fragment thereof.

Preferably, the non-reciprocal t(3;5) translocation comprises the sequence TATACATACATACGGATATATGTATAAAATCATAGGGAGTGAAGTAGTGGCCAAGAAA ACATGCCAG or a variant, homologue deviate or fragment thereof

## CONSTRUCTS

Nucleic acid corresponding to the alteration identified by the methods of the present invention may be cloned and ligated into a construct.

The term "construct" - which is synonymous with terms such as "conjugate", "cassette" and "hybrid" - includes a nucleotide sequence directly or indirectly attached to a promoter.

The construct may contain or express a marker, which allows for the selection of the nucleotide sequence construct in a cell - such as bacterial, plant or mammalian cell. Various markers exist which may be used, for example those encoding mannose-6-phosphate isomerase (especially for plants) or those markers that provide for antibiotic resistance - e.g. resistance to G418, hygromycin, bleomycin, kanamycin and gentamycin.

## VECTORS

Nucleic acid corresponding to the alteration identified by the methods of the present invention may be cloned and ligated into a vector.

The term "vector" includes expression vectors, transformation vectors and shuttle vectors.

The term "transformation vector" means a construct capable of being transferred from one entity to another entity - which may be of the species or may be of a different species. If the construct is capable of being transferred from one species to another e.g. from an *E. coli* plasmid to a bacterium, such as of the genus Bacillus, then the transformation vector is sometimes called a "shuttle vector". It may even be a construct capable of being transferred from an *E. coli* plasmid to an Agrobacterium to a plant.

The vectors may be transformed into a suitable host cell as described below to provide for expression of a polypeptide.

The vectors may be for example, plasmid, virus or phage vectors provided with an origin of replication, optionally a promoter for the expression of the said polynucleotide and optionally a regulator of the promoter.

The vectors may contain one or more selectable marker nucleotide sequences. The most suitable selection systems for industrial micro-organisms are those formed by the group of selection markers which do not require a mutation in the host organism. Examples of fungal selection markers are the nucleotide sequences for acetamidase (*amd*S), ATP synthetase, subunit 9 (*oli*C), orotidine-5'-phosphate-decarboxylase (*pvr*A), phleomycin and benomyl resistance (benA). Examples of non-fungal selection markers are the bacterial G418 resistance nucleotide sequence (this may also be used in yeast, but not in filamentous fungi), the ampicillin resistance nucleotide sequence (*E. coli*), the neomycin resistance nucleotide sequence (*Bacillus*) and the *E. coli uid*A nucleotide sequence, coding for β-glucuronidase (GUS).

Vectors may be used *in vitro*, for example for the production of RNA or used to transfect or transform a host cell.

Thus, polynucleotides may be incorporated into a recombinant vector (typically a replicable vector), for example, a cloning or expression vector. The vector may be used to replicate the nucleic acid in a compatible host cell.

## VARIANTS/HOMOLOGUES/DERIVATIVES/FRAGMENTS

The present invention encompasses the use of variants, homologues, derivatives and fragments thereof.

The term "variant" is used to mean a naturally occurring polypeptide or nucleotide sequences which differs from a wild-type sequence.

The term "fragment" indicates that a polypeptide or nucleotide sequence comprises a fraction of a wild-type sequence. It may comprise one or more large contiguous sections of sequence or a plurality of small sections. The sequence may also comprise other elements of sequence, for example, it may be a fusion protein with another protein. Preferably the sequence comprises at least 50%, more preferably at least 65%, more preferably at least 80%, more preferably at least 85%, more preferably at least 90%, more preferably at least 95%, more preferably at least 96%, more preferably at least 97%, more preferably at least 98%, most preferably at least 99% of the wild-type sequence.

Preferably, the fragment retains 50%, more preferably 60%, more preferably 70%, more preferably 80%, more preferably 85%, more preferably 90%, more preferably 95%, more preferably 96%, more preferably 97%, more preferably 98%, or most preferably 99% activity of the wild-type polypeptide or nucleotide sequence.

The fragment may be a functional fragment.

By a "functional fragment" of a molecule is understood a fragment retaining or possessing substantially the same biological activity as the intact molecule. In all instances, a functional fragment of a molecule retains at least 10% and at least about 25%, 50%, 75%, 80%, 85%, 90%, 95%, 96%, 97%, 98% or 99% of the biological activity of the intact molecule.

The term "homologue" means an entity having a certain homology with the subject amino acid sequences and the subject nucleotide sequences. Here, the term "homology" can be equated with "identity".

In the present context, a homologous sequence is taken to include an amino acid sequence, which may be at least 75, 85 or 90 % identical, preferably at least 95%, 96%, 97%, 98 % or 99% identical to the subject sequence. Although homology can also be considered in terms of similarity (*i.e.* amino acid residues having similar chemical properties/functions), in the context of the present invention it is preferred to express homology in terms of sequence identity.

In the present context, a homologous sequence is taken to include a nucleotide sequence, which may be at least 75, 85 or 90% identical, preferably at least 95%, 96%, 97%, 98 % or 99% identical to the subject sequence. Although homology can also be considered in terms of similarity (i.e. amino acid residues having similar chemical properties/functions), in the context of the present invention it is preferred to express homology in terms of sequence identity.

Homology comparisons may be conducted by eye, or more usually, with the aid of readily available sequence comparison programs. These commercially available computer programs can calculate % homology between two or more sequences.

% homology may be calculated over contiguous sequences, i.e. one sequence is aligned with the other sequence and each amino acid in one sequence is directly compared with the corresponding amino acid in the other sequence, one residue at a time. This is called an "ungapped" alignment. Typically, such ungapped alignments are performed only over a relatively short number of residues.

Although this is a very simple and consistent method, it fails to take into consideration that, for example, in an otherwise identical pair of sequences, one insertion or deletion will cause the following amino acid residues to be put out of alignment, thus potentially resulting in a large reduction in % homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids, a sequence alignment with as few gaps as possible - reflecting higher relatedness between the two compared sequences - will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons. For example, when using the GCG Wisconsin Bestfit package the default gap penalty for amino acid sequences is -12 for a gap and -4 for each extension.

Calculation of maximum % homology therefore firstly requires the production of an optimal alignment, taking into consideration gap penalties. A suitable computer program for carrying out such an alignment is the GCG Wisconsin Bestfit package (University of Wisconsin, U.S.A.; Devereux et al., 1984, Nucleic Acids Research 12:387). Examples of other software than can perform sequence comparisons include, but are not limited to, the BLAST package (see Ausubel *et al.*, 1999 ibid - Chapter 18), FASTA (Atschul et al., 1990, J. Mol. Biol., 403-410), the GENEWORKS suite of comparison tools and CLUSTAL. Both BLAST and FASTA are available for offline and online searching (see Ausubel *et al.*, 1999 ibid, pages 7-58 to 7-60). However, for some applications, it is preferred to use the GCG Bestfit program. A new tool, called BLAST 2 Sequences is also available for comparing protein and nucleotide sequence (see FEMS Microbiol Lett 1999 174(2): 247-50; FEMS Microbiol Lett 1999 177(1): 187-8).

Although the final % homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. GCG Wisconsin programs generally use either the public default values or a custom symbol comparison table if supplied (see user manual for further details). For some applications, it is preferred to use the public default values for the GCG package, or in the case of other software, the default matrix - such as BLOSUM62.

Once the software has produced an optimal alignment, it is possible to calculate % homology, preferably % sequence identity. The software typically does this as part of the sequence comparison and generates a numerical result.

Should Gap Penalties be used when determining sequence identity, then preferably the following parameters are used:

| FOR BLAST | |
|---|---|
| GAP OPEN | 0 |
| GAP EXTENSION | 0 |

| FOR CLUSTAL | DNA | PROTEIN | |
|---|---|---|---|
| WORD SIZE | 2 | 1 | K triple |
| GAP PENALTY | 10 | 10 | |
| GAP EXTENSION | 0.1 | 0.1 | |

For polypeptide sequence comparison the following settings may be used: GAP creation penalty of 3.0 and GAP extension penalty of 0.1. Suitably, the degree of identity with regard to an amino acid sequence is determined over at least 5 contiguous amino acids, determined over at least 10 contiguous amino acids, over at least 15 contiguous amino acids, over at least 20 contiguous amino acids, over at least 30 contiguous amino acids, over at least 40 contiguous amino acids, over at least 50 contiguous amino acids, or over at least 60 contiguous amino acids.

The sequences may also have deletions, insertions or substitutions of amino acid residues, which produce a silent change and result in a functionally equivalent substance. Deliberate amino acid substitutions may be made on the basis of similarity in polarity, charge, solubility, hydrophobicity, hydrophilicity, and/or the amphipathic nature of the residues as long as the secondary binding activity of the substance is retained. For example, negatively charged amino acids include aspartic acid and glutamic acid; positively charged amino acids include lysine and arginine; and amino acids with uncharged polar head groups having similar hydrophilicity values include leucine, isoleucine, valine, glycine, alanine, asparagine, glutamine, serine, threonine, phenylalanine, and tyrosine.

Conservative substitutions may be made, for example, according to the Table below. Amino acids in the same block in the second column and preferably in the same line in the third column may be substituted for each other:

| ALIPHATIC | Non-polar | G A P |
|---|---|---|
| | | I L V |
| | Polar - uncharged | C S T M |
| | | N Q |
| | Polar - charged | D E |
| | | K R |
| AROMATIC | | H F W Y |

The present invention also encompasses homologous substitution (substitution and replacement are both used herein to mean the interchange of an existing amino acid residue, with an alternative residue) may occur i.e. like-for-like substitution - such as basic for basic, acidic for acidic, polar for polar etc. Non-homologous substitution may also occur i.e. from one class of residue to another or alternatively involving the inclusion of unnatural amino acids - such as ornithine (hereinafter referred to as Z), diaminobutyric acid ornithine (hereinafter referred to as B), norleucine ornithine (hereinafter referred to as O), pyriylalanine, thienylalanine, naphthylalanine and phenylglycine.

Replacements may also be made by unnatural amino acids include; alpha* and alpha-disubstituted* amino acids, N-alkyl amino acids*, lactic acid*, halide derivatives of natural amino acids - such as trifluorotyrosine*, p-Cl-phenylalanine*, p-Br-phenylalanine*, p-I-phenylalanine*, L-allyl-glycine*, β-alanine*, L-α-amino butyric acid*, L-γ-amino butyric acid*, L-α-amino isobutyric acid*, L-ε-amino caproic acid#, 7-amino heptanoic acid*, L-methionine sulfone#*, L-norleucine*, L-norvaline*, p-nitro-L-phenylalanine*, L-hydroxyproline#, L-thioproline*, methyl derivatives of phenylalanine (Phe) - such as 4-methyl-Phe*, pentamethyl-Phe*, L-Phe (4-amino)#, L-Tyr (methyl)*, L-Phe (4-isopropyl)*, L-Tic (1,2,3,4-tetrahydroisoquinoline-3-carboxyl acid)*, L-diaminopropionic acid # and L-Phe (4-benzyl)*. The notation * has been utilised for the purpose of the discussion above (relating to homologous or non-homologous substitution), to indicate the hydrophobic nature of the derivative whereas # has been utilised to indicate the hydrophilic nature of the derivative, #* indicates amphipathic characteristics.

Variant amino acid sequences may include suitable spacer groups that may be inserted between any two amino acid residues of the sequence including alkyl groups - such as methyl, ethyl or propyl groups - in addition to amino acid spacers - such as glycine or β-alanine residues. A further form of variation involves the presence of one or more amino acid residues in peptoid form will be well understood by those skilled in the art. For the avoidance of doubt, "the peptoid form" is used to refer to variant amino acid residues wherein the α-carbon substituent group is on the residue's nitrogen atom rather than the α-carbon. Processes for preparing peptides in the peptoid form are known in the art, for example, Simon RJ et al., PNAS (1992) 89(20), 9367-9371 and Horwell DC, Trends Biotechnol. (1995) 13(4), 132-134.

The nucleotide sequences for use in the present invention may include within them synthetic or modified nucleotides. A number of different types of modification to oligonucleotides are known in the art. These include methylphosphonate and phosphorothioate backbones and/or the addition of acridine or polylysine chains at the 3' and/or 5' ends of the molecule. For the purposes of the present invention, it is to be understood that the nucleotide sequences may be modified by any method available in the art. Such modifications may be carried out to enhance the *in vivo* activity or life span of nucleotide sequences useful in the present invention.

KITS

The materials for use in the methods of the present invention are ideally suited for the preparation of kits.

Such a kit may comprise containers, each with one or more of the various reagents (typically in concentrated form) utilised in the methods of the present invention that are described herein, including, for example, buffers and the appropriate nucleotide triphosphates (e.g., dATP, dCTP, dGTP and dTTP; or rATP, rCTP, rGTP and UTP), DNA polymerase and one or more primers for use in the present invention may be also be included.

Oligonucleotides in containers can be in any form, e.g., lyophilized, or in solution (e.g., a distilled water or buffered solution), etc. Oligonucleotides ready for use in the same amplification reaction can be combined in a single container or can be in separate containers.

The kit optionally further comprises a control nucleic acid.

A set of instructions will also typically be included.

FURTHER ASPECTS

In a further aspect, there is provided a method of measuring the copy number frequency of one or more nucleic acid sequences in a sample, comprising the steps of: (a) providing one or more aliquot(s) of the sample, wherein each aliquot comprises nucleic acid in an amount that is less than one genome per aliquot; (b) amplifying one or more nucleic acid sequences in the aliquot(s) in a first amplification reaction; (c) amplifying in a second amplification reaction two or more nucleic acid sequences from the aliquot(s) prepared according to step (b), wherein at least one of the nucleic acid sequences is a test marker and at least one of the nucleic acid sequences is a reference marker; and (d) calculating the copy number of the test marker by comparing the number of amplified products for the test marker with those for the reference marker.

In a further aspect, there is provided a method of measuring the copy number frequency of one or more nucleic acid sequences in a sample, comprising the steps of:

(a) providing one or more aliquot(s) of the sample, wherein each aliquot comprises nucleic acid in an amount that is less than one genome per aliquot; (b) amplifying a test marker in each of the aliquot(s) in a first amplification reaction; (c) dividing each of the amplified products from the first amplification reaction into at least two replica aliquots; (d) amplifying in a second amplification reaction a test marker in at least one of the replica aliquot(s) prepared according to step (c); and (d) calculating the copy number by comparing the number of amplified products from the second amplification reaction for the test marker with that of a reference marker.

In a further aspect, there is provided a method of measuring the copy number frequency of one or more nucleic acid sequences in a sample, comprising the steps of:

(a) providing one or more aliquot(s) of the sample, wherein each aliquot comprises nucleic acid in an amount that is less than one genome per aliquot; (b) amplifying a test marker and a reference marker from each of the aliquot (s) in a first amplification reaction; (c) dividing each of the amplified products from the first amplification reaction into at least two replica aliquots; (d) amplifying in a second amplification reaction the test marker from a first replica aliquot and the reference marker from a second replica aliquot prepared according to step (c); and (e) calculating the copy number by comparing the number of amplified products for the test marker and the reference marker.

In a further aspect, there is provided a a method of measuring the copy number frequency of one or more nucleic acid sequences in a sample, comprising the steps of:

(a) providing one or more aliquot(s) of the sample, wherein each aliquot comprises nucleic acid in an amount that is less than one genome per aliquot; (b) amplifying one or more nucleic acid sequences in each of the aliquot(s) in a first amplification reaction, wherein at least one of the nucleic acid sequences is a test marker and at least one of the nucleic acid sequences is a reference marker; (c) dividing each of the amplified products from the first amplification reaction into at least two replica aliquots; (d) amplifying in a second amplification reaction the test marker from a first replica aliquot and the reference marker from a second replica aliquot prepared in step (c); and (e) calculating the copy number of the test marker by comparing the number of amplified products for the test marker with those for the reference marker.

GENERAL RECOMBINANT DNA METHODOLOGY TECHNIQUES

The present invention employs, unless otherwise indicated, conventional techniques of molecular biology, microbiology, and recombinant DNA technology which are within the capabilities of a person of ordinary skill in the art. Such techniques are explained in the literature. See, for example, J. Sambrook, E. F. Fritsch, and T. Maniatis, 1989, Molecular Cloning: A Laboratory Manual, Second Edition, Books 1-3, Cold Spring Harbor Laboratory Press; Ausubel, F. M. et al. (1995 and periodic supplements; Current Protocols in Molecular Biology, ch. 9, 13, and 16, John Wiley & Sons, New York, N.Y.); B. Roe, J. Crabtree, and A. Kahn, 1996, DNA Isolation and Sequencing: Essential Techniques, John Wiley & Sons; M. J. Gait (Editor), 1984, Oligonucleotide Synthesis: A Practical Approach, Irl Press; and, D. M. J. Lilley and J. E. Dahlberg, 1992, Methods of Enzymology: DNA Structure Part A: Synthesis and Physical Analysis of DNA Methods in Enzymology, Academic Press. Each of these general texts is herein incorporated by reference.

The invention will now be further described by way of Examples, which are meant to serve to assist one of ordinary skill in the art in carrying out the invention and are not intended in any way to limit the scope of the invention. Additionally, each of the various embodiments and aspects of the present invention as delineated hereinabove and as claimed in the claims section below finds experimental support in the following Examples.

EXAMPLES

**Example 1**

Materials and Methods

Molecular Copy-number Counting (MCC)

SK-RC-9 and SK-RC-12 cell lines [20] were cultured in DMEM plus 10% foetal calf serum. Genomic DNA from SK-RC-9 and SK-RC-12 cells was prepared using the Qiagen DNeasy Tissue kit (Qiagen Ltd. UK). DNA was diluted with distilled water to approximately 10 genomes/$\mu$l (about 30 pg/$\mu$l), and stored at -70°C in aliquots.

In outline, assaying of genomic samples for multiple sequences in MCC is carried out using a two-phase PCR amplification, as shown diagrammatically in Figure 1. Three PCR primers (forward and reverse, plus a nested forward-internal primer) are used for each locus (i.e. genomic marker) to be assayed. In the first phase, multiple PCR primer pairs (forward and reverse) are combined in a single multiplex reaction. The products of this reaction are used as the templates for the second-phase PCR reactions, each of which uses the forward-internal and reverse primers for a single sequence. Primer selection was carried out using simple criteria (similar to design for conventional uses of PCR) after masking repetitive elements from the genomic sequence (Assembly NCBI 35, http://www.ensembl.org) using Repeatmasker (http://www.repeatmasker.org). Typically, primer length is 18-20bp, with a Tm between 52-60°C (based on the calculation Tm=2x(A+T)+4x(G+C)). Design requires at least two G or C bases at the 3' end and at least one at the 5' end. No runs of any single base longer than 4 bases are allowed. Internal amplimer length is designed to be between 80-150bp and the position of the external primer no more than 150bp upstream of forward-internal primer.

DNA concentration is important in MCC analysis but need not be too precise as accuracy is obtained with average DNA content of ~02-06 haploid genomes per aliquot. Starting concentration of the genomic DNA preparations was determined using ultra-violet spectrophotometry and based on this, a series of dilutions (conveniently 6) were made that were expected to give between 0.25 and 8 genomes of DNA per sample. Sixteen aliquots of each dilution were assayed (using MCC in 96-well plates, as described above) for each of four markers, corresponding to DNA segments believed to be present at one copy per haploid genome. The proportion of aliquots (wells) which scored positive (averaged across all four markers) was used to calculate the actual DNA concentration in each dilution. These data were in turn used to determine the exact degree of dilution required for MCC analysis. When the working concentration for MCC has been determined, the DNA dilution may be used for all MCC steps. Each new preparation of DNA requires independent titration.

For MCC, a master mix was prepared containing the forward and reverse PCR primers for all sequences to be assayed (0.15$\mu$M of each oligo), 1x Gold PCR buffer (Perkin-Elmer), 2mM MgCl$_2$, 200$\mu$M each dNTP and 0.1u/$\mu$l Taq Gold DNA polymerase (Perkin-Elmer) and about 0.03 genomes/$\mu$l (0.09pg/$\mu$l) of genomic DNA. 10$\mu$l of this mix were dispensed into each of 88 wells of a 96-well plate and the remaining 8 wells (negative controls) received 10$\mu$l of a similar mix but lacking DNA. All samples were overlaid with 20$\mu$l mineral oil. Thermocycling was carried out with hot start at 93°C for 9 minutes, followed by 25 cycles of 20 seconds 94°C, 30 seconds 52°C and 1 minute 72°C. Each PCR reaction was diluted to 500$\mu$l with water, and 5$\mu$l samples used as template in each second-phase (marker-specific) semi-nested PCR (1.5mM MgCl2, 1$\mu$M of the relevant forward-internal and reverse primers, other concentrations as before and thermocycling at 93°C for 9 minutes, followed by 33 cycles of 20 seconds 94°C, 30 seconds 52°C and 1 minute 72°C. After the semi-nested PCR, 8$\mu$l of 2xloading buffer (15% w/v Ficoll 400, 0.1mg/ml bromophenol blue, 4x Sybr Green, 1X TBE) were added to each well and amplification products were analyzed by electrophoresis for 10 minutes at 10V/cm in pre-cast 108-well horizontal 6% polyacrylamide gels (MIRAGE gels; Genetix Ltd., UK) scoring the presence or absence of PCR product in each sample. In later experiments, results were scored by melting-curve analysis using an ABI 7900HT with the manufacture's SDS software.

In these cases, the PCR mixture for the second-phase PCRs was modified to contain 4mM MgCl$_2$ and 0.5x SyBr GreenI (Cambrex, UK). All second-phase PCR reactions were set up robotically in multiple 384-well microtitre plates (each plate containing the 96 reactions for each of four markers). (Note: the second, semi-nested PCR stage could be carried out in a second 96-well plate using a multi-channel pipette for transfers, rather than a robotic system).

Statistical analysis of MCC data

If DNA molecules are distributed randomly amongst a series of aliquots then, from the number of aliquots scoring positive for any given sequence, the concentration of that sequence (expressed in copies per aliquot) can be determined from the Poisson equation (see Supplementary information). If two or more sequences are analyzed in this way on the same set of aliquots of genomic DNA, then their relative concentrations, and hence their elative abundance in the genomic DNA, can be calculated.

For instance, if two DNA markers (A and B) were scored on the same set of 88 aliquots, and if the numbers of aliquots scoring positive for each marker were 34 and 56 respectively, then the average concentrations of the two sequences can be calculated (from equation ii, Supplementary information part A online) as 0.49 and 1.0 copies per aliquot, respectively. Hence, if sequence A is known to be present at n copies per genome, it may be inferred that sequence B is present at 2n copies per genome.

Fluorescence *in situ* hybridization (FISH)

FISH analysis was carried out following standard protocols with whole chromosome paints (Vysis) or BAC DNA (BAC clones from Invitrogen Ltd.) made fluorescent by DIG-nick-translation and the fluorescent antibody enhancer kit for DIG detection (ROCHE Diagnostics Ltd, UK) according to the manufacturer's instructions. BAC clones used in this study of SK-RC-9 were RP11-24E1, RP11-781E19, RP11-413E6 on the short arm of chromosome 3 and CTD-2193G5, CTD-2197I11 on the long arm of chromosome 5. BAC clones used in this study of SK-RC-12 were RP11-328N12, RP11-24E1, RPH-413E6, RP11-528E14, RP11-424C9. BAC clone positions are from the Ensembl Human Genome Server, Assembly NCBI 35.

Filter hybridization analysis

Genomic DNA was digested to completion with restriction enzymes, fractionated on 0.8% agarose gels and transferred to HybondN (Amersham) membranes. Southern filter hybridization was carried out with fragments isolated from plasmid vectors [24], radio-labelled by random oligonucleotide priming reactions as described [25].

The genomic probes for analysis of 5q and 3p in SK-RC-9 DNA were amplified from human DNA by PCR and the products cloned using the TOPO cloning system (Invitrogen) and the sequences verified. Primer sequences for the chromosome 3 probe 3pA were AAGCAGGTTAAGGGAGAAGATGAC (genomic position 74112725 to 74112748 bp) and CTCTGAACTCTCTTATTTAAAG (genomic position 74113146 to 74113167 bp), for the chromosome 3 probe 3pB were CCCATTGCTCCCCAGCC (genomic position 74114114 to 74114137 bp) and GCTGTTGGAGGATGAGAGG (genomic position 74114239 to 74114257 bp) and for the chromosome 5q probe were CTGTTCATTCCTTCAACTTCCTA (genomic position 105386013 to 105386035 bp) and GCTGATTTTATACATATATCTGTATG (genomic position 105386412 to 105386437 bp).

The chromosome 3 probe for filter hybridization of SK-RC12 DNA was made by cloning the genomic PCR product using primers GAATTCAGCTATTCAACGC (genomic position at 81.938.151) and GGAAGTGCTAAACACAATGG (genomic position at 81.938.388).

Inverse PCR cloning

Inverse PCR cloning was carried out as described [21]. SK-RC-9 DNA (5□g) was digested with 100 units XbaI (New England Biolabs) overnight at 37°C. Digested DNA was extracted with phenol and recovered with ethanol precipitation. The digested DNA was circularized by ligation with 3200 units T4 DNA ligase (New England BioLabs) in a volume of 600 µl at 16°C for 16 hours. Ligated DNA was precipitated with ethanol and dissolved in 40 µl Tris-HCl pH 7.4 at 25°C, 1mM EDTA. Five microlitres (~0.6µg DNA template) was used in a 50µl. PCR reaction containing 500µM of each dNTP, 1µM forward and reverse primers, 1 x buffer (Expand Long Template Buffer 2, Roche) and 3.75u Expand Long Template enzyme (Roche). PCR amplification conditions were 94 °C for 2 min, followed by 35 cycles of 94 °C for 15 sec, 60 °C for 30 sec, 68 °C for 15 min, and a final extension step at 68 °C for 30 min. The PCR product was separated on a 1% agarose gel, purified using the QIAquick Gel Extraction Kit (Qiagen) and cloned into the TOPO cloning vector (Invitrogen). The primer sequences for inverse PCR of translocation junction were CTTCCATACCACTTATGGTGTCTA reverse (chromosome 3p genomic position at 74112296 to 74112319 bp) and AATGCAGACCCTCAAACTATACC forward (chromosome 3p genomic position at 74112472 to 74112494 bp).

Primer sequences for PCR amplification of the deletion fusion region of chromosome 3 in SK-RC-12 were 5'-AATGTCATGCAGCATATGAC (genomic position at 81.648.380) and TGATCTTGATTACATAGCATT (genomic position at 81.937.983).

**Example 2**

Molecular Copy-number Counting (MCC)

Normal cells are diploid for autosomal genes while cancer cells may have chromosomal translocations, deletions, amplifications or inversions. If these changes involve copy-number alterations from the diploid state, it is possible to determine this by counting the DNA reiteration frequency. The MCC method achieves this by analysis of the frequency with which PCR amplification of genomic marker occurs at limiting DNA dilution (effectively single DNA molecules). Multiple PCR reactions are performed in a 96-well format and relative copy-number determined for adjacent markers depending on the number of wells with a PCR product.

The essence of the MCC method is described in Figure 1, with an exemplar of a unbalanced translocation. Renal cell carcinomas frequently carry non-reciprocal translocations between chromosomes 3 and 5 (p;q) [4,5,17,18] resulting in an imbalance of copy-number. This is illustrated in Figure 1C where the translocation results in ln copy-number for the distal portion of the black chromosome and 2n proximal to the translocation. Genomic DNA is highly diluted as described for HAPPY mapping [19] and aliquots containing less than one haploid genome of DNA are distributed to 88 wells of a 96-well micro-titre plate, leaving 8 wells for negative PCR controls. The first round of PCR analysis is a multiplexed amplification step for each of the aliquots with all pooled outer primers in each well of the single 96-well plate (Fig. 1D), so that all copies of any target sequence are amplified to some extent. The second PCR round is a semi-nested PCR step for each individual marker (i.e. not multiplexed), using as template the PCR product transferred from the multiplexed plate into fresh replica plates (Fig. 1E, F). These second round PCR products are separated on polyacrylamide gels (Fig.1 G, H) and scored. The proportion of aliquots which are positive for any particular marker reflects the relative copy-number of that marker in the genome. In the experiments shown here, the number of positive PCR reactions were manually counted. The various steps are carried out for convenience employing a robotic transfer system to move samples between micro-titre plates for PCR and semi-nested PCR and from the second micro-titre plate to the gel. This can readily be carried out manually using multi-channel pipettes if robotics are not available.

Examples of gel visualization of the PCR products in MCC applied to renal cell carcinoma are shown in Figure 1G and H, for markers distal or proximal to the non-reciprocal breakpoint respectively. In the former, 24 wells show a PCR product whereas in the latter 46 wells show a product, indicating approximately 2-fold copy-number difference between the markers. (Details of statistical analysis can be found in the supplementary information). When carrying out an initial scan of a chromosomal region, widely-spaced markers may be used and the distance between them can be varied according to need. For instance, if no cytogenetic data are available (such as may be the case with DNA obtained from small biopsy samples), it may be advantageous to scan a whole chromosome before focussing on one area for more detailed study. About 70 markers at about 2Mb spacing would give this information for the whole of chromosome 3.

## Example 3

### Identification and cloning of a non-reciprocal translocation in kidney cancer

One motivation behind the development of the MCC method was to precisely locate the breakpoints of the recurrent non-reciprocal chromosomal translocation t(3;5)(p;q) in renal cell carcinoma as a prelude to their cloning. These important chromosomal translocations have been extensively studied by cytogenetics and three main breakpoints regions have been described on the short arm of chromosome 3 [6]. Non-papillary and papillary renal cell carcinoma cell lines have been established from primary and metastatic tumour material [20]. FISH analysis of the metastatic renal cell carcinoma SK-RC-9 cell line, using chromosome 3 and 5 paints, revealed the presence of a non-reciprocal t(3;5) chromosomal translocation (Supplementary Figure 1 online). Location of the t(3;5) chromosomal translocation breakpoint was initially investigated by FISH using BAC clones from an approximately 3Mb region (73.2 to 75.8Mb) of chromosome 3p (Fig. 2). Since there is no reciprocal derivative chromosome in the renal carcinoma t(3;5), it is not possible to obtain fine mapping of any BAC clone that spans the breakpoint, as BACS either do or do not hybridize to the der(3;5) chromosome. Thus the BAC-FISH analysis of non-reciprocal translocations thus relies on locating BACS flanking the breakpoint. (Fig. 2).

The most proximal BAC clone located telomeric of the t(3;5) breakpoint was RP11-24E1 (at 73256358-73419679bp from the 3p telomere) because this BAC binds to the normal chromosomes 3 but not to the t(3;5) (Fig. 2A). The BAC clone RP11-781E19 (at 74210885-74236089 bp from the 3p telomere) is located just proximal to the breakpoint as it hybridizes to both normal 3 and t(3;5) chromosomes (Fig. 2B). These data located the breakpoint in the SK-RC-9 to a region of at most 1Mb of chromosome 3p13-p12.3 (Fig. 2C *et al.*).

We sought to define the location of the translocation breakpoint on chromosome 3 using multiple rounds of MCC at progressively higher resolution. The FISH data (Fig. 2) showed that chromosome 3 short arm sequences lying telomeric to the breakpoint were expected to be present at two copies per cell (due to the polyploidy of SK-RC-9 cells), whilst those proximal to the breakpoint, or on the long arm, were expected to be present in four copies (i.e. comprising two normal chromosomes 3 and two der 3;5 chromosomes). We performed an initial round of MCC examining the copy number of twelve markers spaced at intervals of 02-0.5Mb over about 3.8Mb in the region of chromosome 3p13-p12.3. The results (Fig. 3, round 1) revealed a two-fold shift in relative copy number between markers located at 73760583 bp and 74333559 bp, defining the putative translocation breakpoint within a window of about 570kb. A subsequent round of MCC was conducted using 12 markers at intervals of about 50kb (Fig. 3, round 2), further refining the putative breakpoint region to within about 40kb. Two further MCC rounds (Fig. 3, round 3 and 4) further localised the copy-number shift to approximately 1-4kb and then to 300bp respectively. In addition, the fourth round of MCC revealed an apparent deletion on the centromeric side of the putative translocation (discussed below).

We confirmed that the MCC data corresponded to genomic alterations using filter hybridizations. A probe was made from the region of chromosome 3p (Fig. 4, probe 3pA) corresponding to the copy-number shift and hybridized to filters carrying restriction digested genomic DNA from the SK-RC-9 cells and a different renal cell line SK-RC-12 whose t(3;5) breakpoint lies proximal to that of SK-RC-9 (AD, GC and THR, unpublished). Rearranged bands were observed with two different restriction enzyme digests of SK-RC-9 (Fig. 4A), showing that the MCC method had identified a genuine abnormality in chromosome 3 in this cell line.

## Example 4

### Cloning the non-reciprocal t(3;5) translocation breakpoint

Round 4 of the MCC analysis of SK-RC-9 DNA showed a copy-number shift that, given its genomic location, was a candidate for the junction of the non-reciprocal translocation between chromosome 3 and 5. Since the normal sequence of this region of chromosome 3p is known, we designed a pair of chromosome 3 primers for inverse PCR cloning of the DNA corresponding to the abnormality. This method for cloning unknown DNA associated with a known DNA segment relies on design of PCR primers which can be extended in opposite directions (illustrated in Fig. 5A) on a circularised DNA template [21]. Accordingly, SK-RC-9 DNA was digested with XbaI, intra-molecular circles made and PCR carried out to produce an ~ 1.5 kb band. The sequence of this PCR product showed that it comprised the junction of the t(3;5) non-reciprocal chromosomal translocation (Fig. 5B) in which a region of chromosome 5q (the location is shown in Fig. 5C, co-ordinate 105386443bp) had fused with a region of chromosome 3p (Fig. 5D, co-ordinate 74111893bp). The adenine residue at the junction may derive from either chromosome. The rearrangement of this chromosome 5 segment in DNA from SK-RC-9 cells was formally shown using filter hybridization studies (Fig. 3B, probe 5q).

**Example 5**

MCC can detect cryptic chromosomal changes

During the definition of the translocation breakpoint by the MCC analysis, we observed an additional copy-number reduction over a region covering about 700bp just centromeric of the breakpoint (Fig. 3 round 4). This finding was verified with two independent MCC experiments (Supplementary Figure 2) comparing SK-RC-9 DNA to another renal carcinoma (SK-RC-12) with a t(3;5) located in a different 3p cluster region.

A possible explanation for this anomaly in SK-RC-9 DNA could be a small deletion on the t(3;5) chromosome, just centromeric of the translocation breakpoint. We sought to substantiate this by genomic PCR with primers flanking the region. While we could amplify a fragment of the expected size (907bp) from normal chromosome 3, there was no evidence of a smaller product that should have been amplified across the deleted segment of DNA (data not shown). Thus, this short deletion must be accompanied by insertion of sequences from another location. The filter hybridization analysis in Figure 4 confirms this possibility. When SK-RC-9 DNA is digested with BgIII, we would expect a rearranged fragment from the der(3;5) chromosome of about 4.3kb when hybridized with the 5q probe if the translocation were simply associated with a small deletion. Instead we observed a larger fragment of around 5kb (Fig. 4B). Further, the observed sizes of NcoI and SacI fragments using the 3pB probe (Fig. 4C) are both significantly larger than expected for a simple deletion (observed 11.5kb or 9.5kb and expected 4.5kb or 4kb respectively). The NcoI hybridization data is especially significant because the 3pB probe does not detect the translocation junction but only the additional abnormality. These data suggest an insertion together with the deletion and that the insertion accompanying the 3p micro-deletion seems to be greater than 7kb.

**Example 6**

A 289kb deletion on chromosome 3 in a kidney cancer cell line detected by MCC

A further exemplification of the sensitivity of MCC was provided by the characterisation of a cryptic deletion in of the clear cell renal carcinoma SK-RC-12 cell line which carries a non-reciprocal t(3p;5q) translocation (data not shown). FISH analysis with BAC clones RP11-528E14 (chromosome 3 at 76.7 - 76.9 Mb) and RP11-424C9 (chromosome 3 at 87.7 - 88.0 Mb) delineated the breakpoint region of the t(3;5) to a large region of around 10Mb. MCC analysis was performed using a panel of markers spanning this region at intervals of about 0.25Mb (Fig. 6A, round 1). A copy number shift was observed between markers 22 and 23 (the analytical gels for PCR products of makers 21, 22, 24 and 25 are shown in Supplementary Fig. 3). Subsequent rounds of MCC with more closely-spaced markers (Fig. 6A, round 2 and 3) resolved the region to about 2kb and a final round of MCC localised the copy number shift to within 400bp (Fig. 6A, round 4). The presence of a genomic alteration was confirmed by filter hybridization using a 237bp probe from chromosome 3 and comparing the restriction fragments in SK-RC-12 DNA with those of a lymphoblastoid cell line (LCL). A rearranged fragment was observed in each case with SK-RC-12 DNA (Supplementary Fig. 4)

The genomic region corresponding to the round 4 copy number shift was obtained using inverse PCR. To obtain this junction region sequence, genomic DNA was digested with NcoI and self-ligated to form circular DNA templates, amplified using the chromosome 3 sequence located with the MCC round 4. The PCR product was cloned and the sequence obtained, revealing that the copy number change resulted from a simple deletion of ~289 kb chromosome 3p (Fig. 6B, 81.64 and 81.94 Mb). The sequences of the regions flanking the deletion point from normal chromosome 3 are compared to the fused chromosome 3 in SK-RC-12 (Fig. 6B). This discloses the identity of a 6bp region on both ends of the deletion segment (Figure 6B) suggesting that this micro-homology may have been used in non-homologous end joining to repair the double strand breaks.

**Example 7**

Statistical analysis of MCC results

If DNA molecules are distributed randomly amongst N aliquots to give an average of Z molecules per aliquot then, according to the Poisson distribution, the number of aliquots Np which are expected to contain at least one molecule of the DNA is given by:

$$Np=N(1-e^{-z}) \qquad (i)$$

Conversely, if a panel of N sub-genomic aliquots of DNA is tested by PCR for a given sequence, and if Np of these aliquots score positive for the sequence, (i.e. contains at least one copy of the sequence) then the average number of molecules of that sequence per aliquot can be calculated as:

$$Z= -\ln(1-Np/N) \qquad (ii)$$

Hence, from the number of aliquots scoring positive for any given sequence, the concentration of that sequence (expressed in copies per aliquot) can be determined. If two or more sequences are analysed in this way on the same set (or similar sets) of aliquots of genomic DNA, then their relative concentrations and hence their relative abundance in the genomic DNA can be calculated.

**Example 8**

BAC clones and PCR primers used for MCC analysis

BAC clones

BAC clones used to define the position of the t(3;5) in SK-RC-9 cells were located on chromosome 3 and 5 using the Ensembl Human Genome database, assembly NCBI 35 at http://www.ensembl.org/

List of BAC clones

BAC coordinates on 3p (where residue 1 is located at chromosome 3p telomere):

RP11-24E1 73256358-73419679 bp
RP11-781E19 74210885-74236089 bp
RP11-413E6 75698634-75885406 bp
BAC coordinates on 5q (where residue 1 located at chromosome 5 centromere)
CTD-2193G5 102719818-102903604 bp
CTD-2197111 108078031-108252168 bp

Primers used in the MCC analysis of SK-RC-9

The primers were derived from the Ensembl Human Genome Server, Build 35 and the co-ordinates given refer to that sequence information. As this is under ongoing revision, the precise location at any future time should be determined using a BLAST search of the genome database.
The primers used analysis of SK-RC-9 are shown in Table 1.

List of BAC clones used for SK-RC-12 FISH

BAC coordinates on 3p (where residue 1 is located at chromosome 3p telomere):

RP11-328N12 chromosome 3 at 72.5 - 72.7 Mb
RP11-24E1 chromosome 3 at 73.1 - 73.3 Mb
RP11-413E6 chromosome 3 at 75.5 - 75.7 Mb
RP11-528E14 chromosome 3 at 76.7 - 76.9 Mb
RP11-424C9 chromosome 3 at 87.7 - 88.0 Mb

DISCUSSION

The t(3,5) translocation of kidney cancer

We have shown that MCC can directly count the copies of different sequences whilst scanning a chromosomal or genomic region to identify local variations in copy-number. Iterations of this method allow the precise boundaries of the aberrant segment to be located rapidly, as we have shown by locating and cloning the breakpoint of a non-reciprocal chromosomal translocation. The specific breakpoint that we have cloned represents the first example of cloning a *de novo* non-reciprocal chromosomal translocation. Kidney cancer has a very poor prognosis [22] and tumours arising in the proximal tubule (non-papillary kidney cancer) often have a non-reciprocal chromosomal translocation t(3;5). The breakpoints on chromosome three cluster to three different regions of the short arm [6] and the one we describe in this paper (in SK-RC-9 cells) locates at the most distant cluster (at chromosome 3p13). The analysis of the breakpoint DNA sequence from either chromosome 3p or 5q in SK-RC-9 shows that there is no loss or gain of material specifically at the junction, implying precise end breakage and repair. In addition, the breaks do not involve cleavage within any known or putative genes (see Fig. 5). Thus, the translocation would not to yield a fusion gene, and rather suggesting a different mechanism for the main oncogenic outcome of the non-reciprocal translocation. The use of MCC to analyse and clone the breakpoints of other renal carcinoma non-reciprocal translocations will shed more light on this, and comparison of breakpoint sequences between different translocations should help clarify whether there is any sequence-specific mechanism of translocation.

Chromosomal alterations co-exist with non-reciprocal translocations in kidney cancer

In this study, the new MCC technology has been used to determine the location of a non-reciprocal translocation breakpoint and the existence of twp cryptic deletions on chromosome 3. This efficacy of the technique in using iterative rounds of copy number determination with increasing resolution has been applied to clone and sequence two of the cancer-associated changes. There is increasing evidence that deletion and amplification accompanies chromosomal translocations, but whether it is functionally significant or not has been hard to determine due to paucity of functional tests. As the chromosomes involved in inter-chromosomal translocations are inherently unstable at the time of double-strand breakage, the incidence of additional changes may not seem too surprising. However, DNA repair mechanisms inherently have high fidelity, adding credence to the notion that changes can be functionally important.

The MCC approach to genome analysis

MCC has several advantages over other methods for locating alterations in copy-number. The method offers effectively unlimited resolution as sequences can be examined from wide intervals down to a few hundred base pairs. Since MCC utilises genome sequence information, it only requires a genome database for its operation and a series of PCR primers. Libraries of PCR primers, formatted for use in MCC, may be established to enable the rapid scanning of chromosomal regions or complete genomes. Further, the method should be applicable to regions of genomic amplifications, as well as to deletions.

Unlike array-based technologies for copy-number determination, MCC does not require whole genome amplification or any hybridization step. This obviates any problems that might arise from biased amplification, incomplete suppression of repeat sequences within the probe or cross-hybridization, as can occur when using short oligo arrays or through amplification of E. coli DNA contaminating BAC/PACs for arrayCGH [9]. Accurate copy number quantitation by MCC depends upon successful amplification of all of the copies of a locus in the panel of aliquots. Most PCR primer sets either work well (detecting most or all copies) or not at all (detecting no copies, or giving very poor PCR products in all cases). The latter are obvious and can be discarded from the analysis, as was done for markers 5 and 7 (Fig. 3, round 1). Nevertheless, a single marker of apparently low copy-number must be viewed with caution, as it could arise solely from a failure to detect some of the copies of that locus. In practice, this is not a handicap, since the apparent loss of copy will be confirmed (or not) as the analysis proceeds to higher resolution. MCC is essentially a digital approach which simplifies interpretation of results whereas the micro-array approaches are quantitative often require complex algorithms for interpretation [23]. Although MCC is easily applicable to manual operation, it also lends itself well to automation, and should be adaptable to other platforms for high-through-put PCR analysis with potential savings in time and costs.

MCC requires minuscule amounts of genomic DNA, being applicable to as few as tens to hundreds of cells, and the DNA does not have to be of high molecular weight. We therefore suggest that MCC will enable hitherto impractical studies, such as the detailed analysis of pre-neoplastic biopsy material from patients, the retrospective analysis of archival tumour samples, or the exploration of genomic variability across different small regions of a tumour. MCC should also simplify the analysis of hereditary chromosomal abnormalities that affect copy-number, whether associated with disease or forming part of a normal spectrum of human variation. The use of MCC in our current work employs cell lines where the imbalances are constant from cell to cell. The application of MCC to constitutional copy number differences (e.g. inherited syndromes), will be similar since all the DNA will be identical. This will not necessarily be the case of biopsies of disease-based material, where the 'equality' of the sampling could be influential in usefulness (as with other copy number-based methods). Tumour samples may be a particular issue as resections will comprise cancer cells, stromal cells (probably normal karyotype) and inflammatory cells. Nevertheless, the small amounts of material required for MCC and the sensitivity of the approach should enable copy-number anomalies to be detected even against some background of normal DNA.

**TABLE 1:** Primers used in the MCC analysis of SK-RC-9 The primers were derived from the Ensembl Human Genome Server, Build 35 and the co-ordinates given refer to that sequence information. As this is under ongoing revision, the precise location at any future time should be determined using a BLAST search of the genome database.

*The primers used for MCC round 1:*

| marker | location of Ext F (bp) | Ext F | Int F | Common R |
|---|---|---|---|---|
| 1 | 71640850 - 71640869 | GCCAAAGTAGTCATGATGGG | GTGAGCTATGAGCTGTTGC | CTGCAGAGTGATACCTGCC |
| 2 | 71887859 - 71887876 | GTTCGAGGATTGGGAGGG | GCTTGTGCTTTGAGAAGCC | CAGCACAGCTTAACCTAGC |
| 3 | 71914076 - 71914096 | GAAGGAAGAGTAACATAAGGC | CAAGCATCTTGGTCTGTCC | GCATGAAACACTCCAGACC |
| 4 | 72576261 - 72576280 | GGAGAAGTGAGTTTGACAGG | CTTTGTGATACTGGTTACTGC | GCACCAAGAGAAGCTGCG |
| 5 | 72980058 - 72980077 | GTCTGACTTCAAGTTCTACG | CAAGCCATACTTTCTCAGGC | CTCACTGGTGCCAAACAGG |
| 6 | 72953209 - 72953227 | GAGCTCTGGTTTCATGAGG | CCCATGTTGTCTTTCAGTGG | GGGAAACCTACCGTCACC |
| 7 | 73583035 - 73583053 | GGATGTGAGCCAGTTTCGG | CAGCATAGGATGTCATCTGG | GCTTGCCTGTTAACATAGCC |
| 8 | 73760583 - 73760602 | CGCAATTCTTGTTCTTCTGC | CCTACTGTGATAACTCATCC | GATCAGTATGTTCAACATAGG |
| 9 | 74333559 - 74333577 | CAGGGTCAAGGATTCCACC | CCAGTAACACAGTGTAGAGG | GTGTCAGCAGTCTTAGGC |
| 10 | 74650773 - 74650792 | CACCAAACAGGAACAATGGC | GTCATGGACAACATATGCC | GAGTTCACAGTCAGTCTGG |
| 11 | 74667410 - 74667428 | GGTGGAGACTGAGAACAGG | GAGCACATCCAACTCAGCC | CTTGGATTCAAGGAACAACC |
| 12 | 75351795 - 75351813 | CCAGTGGGAACATCATGGC | CTTCTTGCACACTTCAAGG | CTGGATGGGTTCTAGCAGC |

*Primers used for MCC round 2:*

| marker | location of Ext F (bp) | Ext F | Int F | Common R |
|---|---|---|---|---|
| 1 | 73494083 - 73494102 | GAGTCAAGATTGTGCGTTGG | CCTTGAGCAGAGTTGAACC | GGCAGGAGAATGAGTGAGC |
| 2 | 73519165 - 73519184 | GGTGAGTTCATGATTCCTCC | GTTTCTGAATCAGATTACTTGG | CAGGAGAGCCTTCCCAGG |
| 3 | 73575912 - 73575930 | CTGTCTCCTGCTATCCTGC | CTGCTTTGTGACTGACATCC | CATGGGATTGGAAGGATGG |
| 4 | 73622018 - 73622036 | CTCTCCCTCTGAAAGGTGG | GAGCCTGCTTTCCCTTGG | CACAGTGTGATTTCTCTTCG |
| 5 | 73682682 - 73682701 | GCAGATTTGTTGGAACAAGG | CTGGAGAAGCAGAATGTTGC | CCCTGAAAGCATCCAGCG |
| 6 | 73729361-73729380 | CTAGCTCAAAGCAGAACAGC | CACCAAAGGCAAGCCTCC | CACTCCTCTGATGCAACTCC |
| 7 | 73787925 - 73787943 | CTCAGACACAGTACTGACC | GACTCAGAAAGAGTGGTCC | CTCACTGCAGGGAGCAGG |
| 8 | 73826179 - 73826198 | GGGTTAAGTATCCAGTCTCC | GACAACTTGACAATGCATCC | CCACAAGAGTAGACTGAGG |
| 9 | 73874968 - 73874988 | CAACTCTTGAATGCAGATACC | CTTCAGAAAGTCCAAACTGG | GAGAGCCTTTCTAGTAAACC |
| 10 | 73919207 - 73919225 | GACACATGATTCTTCACCC | CCCTCACATTTGGTCTTGG | GTTCGAGACTATGCTGTACC |
| 11 | 73987453 - 73987473 | CTTACTGTGAATTGGAAAGGC | CTGTCGTCTGTCTACTTCC | CACCATTCACTGTAGGACC |
| 12 | 74026256 - 74026275 | GTTTGAAAGCAATCACCAGG | GGAAATGAAAGGCAAAGATGG | GTGTGGATTGAAGTAACTCC |
| 13 | 74081562 - 74081579 | GGCTGCAGAAACCCAGGG | CTGCTATGATATCTACTAGC | GCTCACAACATAAGGAAGG |
| 14 | 74120228 - 74120246 | CCCTCACACCATTCAACCC | GACTGTTACCGTTTCATGGC | GATGGTAGTGTTAGTTTGAGG |

*The primers used for MCC round 3:*

| marker | location of Ext F (bp) | Ext. F | Int. F | Common R |
|---|---|---|---|---|
| 1 | 74094972 to 74094992 | GGCAAATCATTTGATTCCAGG | CTGGGTTTCACTTGAGTAGG | CCTTCTATGTGTTAGACATCG |
| 2 | 74095840 to 74095858 | GGAGCCTGATGAAAGATGG | CTGGCAGAAAGGAAGAAGC | CAGACATACTCTCAACAAAGG |
| 3 | 74097058 to 74097079 | CTTACTCTATTCTACGACAAGC | GCTGCTTTACAAATCTGGC | CCCAATGGCTCCAGACGG |
| 4 | 74103474 to 74103493 | GTACAATTCAAATGCAGTCC | GACTGCATGGCAAGATAGC | CTGCATAGTCTCCCAAAGC |
| 5 | 74108031 to 74108049 | CAGCTACTTCATCTCAGCC | GTGTCAGTAGAAAGCCTTCC | GTTTCTCCTTCTTTGAAGTGC |
| 6 | 74109465 to 74109486 | GAACAACTTTCTCTTGAAAGCC | GAATCTTATGTTCATTCTTCC | CCATCTATGTGCAGCAAGG |
| 7 | 74110081 to 74110100 | GCACTAGTGTGACTTGTACC | GCCTTGAAAGATGTCTCTGC | CCAGTGTTGAAGCAAAGCC |
| 8 | 74111435 to 74111452 | CTCCCACATGGACTGACC | GCACCACATCCTTCCTTGC | GCAGAGGTAGGCAAAGTGG |
| 9 | 74114795 to 74114814 | GAGTGTTGCACTTCTGTTGC | GCACATGACTAGTCCTGGC | CTGTGTATGTAGAAGAAGCC |
| 10 | 74118287 to 74118307 | GTAGAACCTATTCAAATCTCC | CATACATTCTATTGCCATGGC | GCAAGTCACAGAGCCTTGG |
| 11 | 74120228 to 74120246 | CCCTCACACCATTCAACCC | GACTGTTACCGTTTCATGGC | GATGGTAGTGTTAGTTTGAGG |
| 12 | 74333559 to 74333577 | CAGGGTCAAGGATTCCACC | CCAGTAACACAGTGTAGAGG | GATCAGTATGTTCAACATAGG |
| 13 | 74650773 to 74650792 | CACCAAACAGGAACAATGGC | GTCATGGACAACATATGCC | GTGTCAGCAGTCTTAGGC |
| 14 | 86308495 to 86308513 | CCTTGAGCTGTTCCAACCC | GCTGTCTCACTCAGTTGCC | GATGGTCATGATTCCCAAGC |

*The primers used for MCC round 4:*

| marker | localisation Ext F (bp) | ExtF | Int F | Common R |
|---|---|---|---|---|
| 1 | 74109465 - 74109486 | GAACAACTTTCTCTTGAAAGCC | GAATCTTATGTTCATTCTTCC | CCATCTATGTGCAGCAAGG |
| 2 | 74111435 - 74111452 | CTCCCACATGGACTGACC | GCACCACATCCTTCCTTGC | GCAGAGGTAGGCAAAGTGG |
| 3 | 74111449 - 74111467 | GACCTCTTTGCCCAAAAGC | CTTGCTCTCATGCTTAAGCC | CTGAGTGCAGAGGTAGGC |
| 4 | 74111627 - 74111645 | CTGAGTGGTATACATCTGG | GTCCAATAGGAGAAATAAG | CAAAGGCTAATTTCTCCACA |
| 5 | 74111845 - 74111863 | GAGCTGGCTGTAGAATGGG | GGTTCTGGCAAGAGCAGG | CCCACTTTACACTTTAGGC |
| 6 | 74111880 - 74111898 | GCAGGGAGAATACATAGGG | CCAAGAAAACATGCCAGCG | CACATATGAAATCCTTAGCC |
| 7 | 74112175 - 74112194 | CTTCAATACTTACCTCAACC | CTATGATGTAGATGTTTTGTCC | CCTTCCATACCACTTATGG |
| 8 | 74112258 - 74112277 | CAGTAAGACACAAAGATGGG | CACCATAAGTGGTATGGAAGG | CAAATGACTCTGCCTCTGC |
| 9 | 74112473 - 74112491 | ATGCAGACCCTCAAACTAT | GAAATGGCCTTATTTGATACA | AACTGTTGAATCCACCTAC |
| 10 | 74112591 - 74112608 | GGGAGACCTGTAAGATGG | GGTGTCAGGGCCAATGGC | GGTCATCTTCTCCCTTAACC |
| 11 | 74112746 - 74112764 | GACCTGAGTTTTGAGTGCC | GAGATGGGTTCATGTGAGG | CATCAAAAGTGATAGTTAACCC |

(continued)

EP 2 002 016 B1

| *The primers used for MCC round 4:* | | | | |
|---|---|---|---|---|
| **marker** | **localisation Ext F (bp)** | **ExtF** | **Int F** | **Common R** |
| 12 | 74112780 - 74112798 | GAGATGGGTTCATGTGAGG | CTAGAGGCAGATGCTGGC | GGCATTGTGTCTGTGACG |
| 13 | 74113016 - 74113034 | CACAGACACAATGCCAAGC | CATGCATTGTTTCATATGTTCC | GAGCAGGAAGCAGAATGCC |
| 14 | 74113219 - 74113239 | GTAGGTGTATGTGTTATCTCC | CTTCCAGGGGCATTCTGC | CAGATGCCGGAACTCAGC |
| 15 | 74113371 - 74113388 | GGAAGCAGAAAGGAGAGC | GCATCCACAGCCATCTGC | GCTATGAATACCATCATGGG |
| 16 | 74113519 - 74113538 | CCCATGATGGTATTCATAGC | CTACCTTGTCGTTTAGAACC | GTGTGAATAGGGTGTAGAGG |
| 17 | 74113706 - 74113724 | TTGTAATGATCTCCTTTGC | GCCTCCTCAAATTAACCTA | GATAAGATCTTGGGATCTGG |
| 18 | 74113824 - 74113843 | CTCACAGAACAGGAAGTAGC | CTCCTTGTGTTATGGAAGG | CCAATACCCAGGAATGAGC |
| 19 | 74114080 - 74114097 | CAGAGCAGCTTAAGTTCC | CCCATTGCTCCCCAGCC | GCTGTTGGAGGATGAGAGG |
| 20 | 74114239 - 74114257 | CCTCTCATCCTCCAACAGC | CTGATACACATGAGAATGTGC | CTGCCCCTTGTTTCTTAGC |
| 21 | 74114417 - 74114436 | CTCTAACTCTAAGGTAAACT | AGATGGTCAACATTGAAGA | GAGTCTTACTTTAAGCCAT |
| 22 | 74114511- 74114529 | GGTCAACATTGAAGAGTGG | GTAAGACTCAAAAGAAACTGG | CAGAAGTGCAACACTCTGC |
| 23 | 74114795 - 74114814 | GAGTGTTGCACTTCTGTTGC | GCACATGACTAGTCCTGGC | CTGTGTATGTAGAAGAAGCC |
| 24 | 74118287 - 74118307 | GTAGAACCTATTCAAATCTCC | CATACATTCTATTGCCATGGC | GCAAGTCACAGAGCCTTGG |
| Footnote:<br>Ext F = external forward primer used in the first PCR reaction for multiplex PCR<br>Common R = common reverse primers (for each marker) used in both the PCR steps<br>Int. F = internal, semi-nested forward | | | | |

**REFERENCES**

1. Albertson, D.G., Collins, C., McCormick, F. & Gray, J.W. Chromosome aberrations in solid tumors. Nat Genet 34, 369-376 (2003).

2. Rabbitts, T.H. Chromosomal translocations in human cancer. Nature 372, 143-149 (1994).

3. Huntly, B.J., Bench, A. & Green, A.R. Double jeopardy from a single translocation: deletions of the derivative chromosome 9 in chronic myeloid leukemia. Blood 102, 1160-1168 (2003).

4. Kovacs, G., Szucs, S., De Riese, W. & Baumgartel, H. Specific chromosome aberration in human renal cell carcinoma. Int. J. Cancer 40, 171-178 (1987).

5. Kovacs, G. & Brusa, P. Recurrent genomic rearrangements are not at the fragile sites on chromosomes 3 and 5 in human renal cell carcinomas. Hum Genet 80, 99-101 (1988).

6. Kovacs, G. et al. The Heidelberg classification of renal cell tumours. J. Pathol. 183, 131-133 (1997).

7. Pinkel, D. & Albertson, D.G. Comparative genomic hybridization. Annu Rev Genomics Hum Genet 6, 331-354 (2005).

8. Fiegler, H. et al. DNA microarrays for comparative genomic hybridization based on DOP-PCR amplification of BAC and PAC clones. Genes Chromosomes Cancer 36, 361-374 (2003).

9. Menten, B. et al. arrayCGHbase: an analysis platform for comparative genomic hybridization microarrays. BMC Bioinformatics 6, 124 (2005).

10. Brennan, C. et al. High-resolution global profiling of genomic alterations with long oligonucleotide microarray. Cancer Res 64, 4744-4748 (2004).

11. Carvalho, B., Ouwerkerk, E., Meijer, G.A. & Ylstra, B. High resolution microarray comparative genomic hybridisation analysis using spotted oligonucleotides. J Clin Pathol 57, 644-646 (2004).

12. Lucito, R. et al. Representational oligonucleotide microarray analysis: a high-resolution method to detect genome copy number variation. Genome Res 13, 2291-305 (2003).

13. Sebat, J. et al. Large-scale copy number polymorphism in the human genome. Science 305, 525-528 (2004).

14. Jobanputra, V. et al. Application of ROMA (representational oligonucleotide microarray analysis) to patients with cytogenetic rearrangements. Genet Med 7, 111-118 (2005).

15. Zhou, W. et al. Counting alleles reveals a connection between chromosome 18q loss and vascular invasion. Nat Biotechnol 19, 78-81 (2001).

16. Zhao, X. et al. Homozygous deletions and chromosome amplifications in human lung carcinomas revealed by single nucleotide polymorphism array analysis. Cancer Res 65, 5561-5570 (2005).

17. Heim, S. & Mitelman, F. Cytogenetics of solid tumours. Recent Advances in Histopathology, 37-66 (1991).

18. Hoglund, M. et al. Dissecting karyotypic patterns in renal cell carcinoma: an analysis of the accumulated cytogenetic data. Cancer Genet Cytogenet 153, 1-9 (2004).

19. Dear, P.H. & Cook, P.R. Happy mapping: a proposal for linkage mapping the human genome. Nucleic Acids Res 17, 6795-6807 (1989).

20. Ebert, T., Bander, N.H., Finstad, C.L., Ramsawak, R.D. & Old, L.J. Establishment and characterisation of human renal cancer and normal kidney cell lines. Cancer Res. 50, 5531-5536 (1990).

21. Suzuki, T. et al. New genes involved in cancer identified by retroviral tagging. Nat Genet 32, 166-174 (2002).

22. Cohen, H.T. & McGovern, F.J. Renal-cell carcinoma. N Engl J Med 353, 2477-2490 (2005).

23. Daruwala, R.S. et al. A versatile statistical analysis algorithm to detect genome copy number variation. Proc Natl Acad Sci USA 101, 16292-16297 (2004).

24. LeFranc, M.-P., Forster, A., Baer, R., Stinson, M.A. & Rabbitts, T.H. Diversity and rearrangement of the human T cell rearranging γ genes: Nine germ-line variable genes belonging to two subgroups. Cell 45, 237-246 (1986).

25. Feinberg, A.P. & Vogelstein, B.A. A technique for radiolabelling DNA restriction endonuclease fragments to high specific activity. Anal. Biochem. 132, 6-13 (1983).

Various modifications and variations of the described methods and system of the invention will be apparent to those skilled in the art. Although the invention has been described in connection with specific preferred embodiments, it should be understood that the invention as claimed should not be unduly limited to such specific embodiments. Indeed, various modifications of the described modes for carrying out the invention which are obvious to those skilled in molecular biology or related fields are intended to be within the scope of the following claims.

**Claims**

1. A method of measuring the copy number frequency of one or more nucleic acid sequences in a sample, comprising the steps of:

   (a) providing a plurality aliquot(s) of the sample, wherein each aliquot comprises nucleic acid in an amount that is less than one genome per aliquot;
   (b) amplifying one or more nucleic acid sequences in each of the aliquot(s) in a first amplification reaction;
   (c) subdividing one or more of the amplified products into replica aliquots and performing a second amplification reaction for one or more nucleic acid sequences in each of the aliquot(s), wherein at least one of the nucleic acid sequences is a test marker and at least one of the nucleic acid sequences is a reference marker; and
   (d) calculating the copy number by comparing in each of the replica aliquots the number of amplified products obtained for the test marker with the number of amplified products obtained for the reference marker.

2. A method according to claim 1, wherein each aliquot in the first amplification reaction comprises about 0.1-0.9 genomes of DNA per amplification reaction.

3. A method according to claim 1 or claim 2, wherein the copy number of the test marker is calculated by manually counting the number of amplification products for the test marker and the reference marker.

4. A method according to any of the preceding claims, wherein the copy number of the test marker is calculated using the equation:

$$Np=N(1-e^{-z})$$

   wherein N is the number of aliquots; Z is the average number of amplified products per aliquot; and Np is the number of aliquots which are expected to contain at least one molecule of the nucleic acid according to Poisson distribution.

5. A method according to any of claims 1-3, wherein the copy number of the test marker is calculated using the equation:

$$Z= -\ln(1-Np/N)$$

   wherein N is the number of aliquots of nucleic acid tested for a given sequence; Np is the number of aliquots that score positive for the nucleic acid, (i.e. at least one copy of the nucleic acid sequence is amplified).

6. A method according to any of the preceding claims, wherein the amplification reactions are performed using PCR.

7. A method according to any of the preceding claims, wherein the first amplification reaction is performed using forward and reverse primer pairs.

8. A method according to any of the preceding claims, wherein the second amplification reaction is performed using forward-internal and reverse primers.

9. A method according to any of the preceding claims, wherein the concentration of nucleic acid in the sample prior to aliquoting is determined by amplifying one or more nucleic acids believed to be present at only one copy per haploid genome at two or more different dilutions, wherein the proportion of samples at each dilution found positive for the one or more nucleic acids is used to refine the estimate of the DNA concentration and hence determine the dilution required for the subsequent analysis.

10. A method of identifying one or more alterations in a sample of nucleic acid, comprising the steps of:

(a) measuring the copy number frequency of one or more nucleic acid sequences in a first sample and a second sample according to the method of any of claims 1-9;

(b) optionally iteratively repeating the method at progressively higher resolutions for each of the samples; and

(c) identifying one or more differences in the copy number frequency of one or more nucleic acid sequences in the first and second samples.

11. A method according to claim 10, wherein the samples are or are derived from diseased and non-diseased subjects.

12. A method according to claim 11, wherein the disease is cancer.

13. A method according to any of claims 10-12, wherein the method is initially performed at a resolution of 2Mb progressively decreasing to 100 base pairs or less.

14. A method according to any of claims 10-13, wherein the alteration is a translocation, an amplification, a duplication or a deletion.

15. A method of diagnosing a disease in a subject, comprising the steps of:

(a) measuring the copy number frequency of one or more nucleic acid sequences in a sample according to the method of any of claims 1-9; and

(b) comparing the copy number of the one or more nucleic acid sequences with the normal copy number of the one or more nucleic acid sequences;

wherein a difference between the copy numbers of the one or more nucleic acid sequences in the sample and the normal copy number of the one or more nucleic acid sequences is indicative that the subject is suffering from the disease.

16. A method according to claim 15, wherein if the copy number of one or more nucleic acid sequences in the sample of nucleic acid from the subject is greater than the normal copy number is indicative of a translocation, an amplification or a duplication.

17. A method according to claim 15, wherein if the copy number of one or more nucleic acid sequences in the sample of nucleic acid from the subject is less than the normal copy number is indicative of a deletion.

18. A method for cloning one or more alterations in a sample of nucleic acid comprising the steps of:

(a) identifying one or more alterations in a sample of nucleic acid according to any of claims 10-14; and

(b) cloning the one or more alterations.

19. A method according to claim 18, wherein the alteration is cloned using inverse PCR cloning.

**Patentansprüche**

1. Verfahren zum Messen der Häufigkeit der Kopienzahl einer oder mehrerer Nukleinsäuresequenzen in einer Probe, welches die folgenden Stufen umfaßt:

(a) Bereitstellen einer Mehrzahl eines Aliquots (von Aliquots) der Probe, wobei jedes Aliquot Nukleinsäure in einer Menge umfaßt, die kleiner ist als ein Genom pro Aliquot,

(b) Vervielfältigen einer oder mehrerer Nukleinsäuresequenzen in jedem Aliquot (der Aliquots) in einer ersten Vervielfältigungsreaktion,

(c) Aufteilen eines oder mehrerer der vervielfältigten Produkte in Replica-Aliquots und Durchführen einer zweiten Vervielfältigungsreaktion für eine oder mehrere Nukleinsäuresequenzen in jedem Aliquot (der Aliquots), wobei wenigstens eine der Nukleinsäuresequenzen ein Testmarker ist und wenigstens eine der Nukleinsäuresequenzen ein Referenzmarker ist, und

(d) Berechnen der Kopienzahl durch Vergleichen der Anzahl von vervielfältigten Produkten, die für den Testmarker erhalten wurden, mit der Anzahl von vervielfältigten Produkten, die für den Referenzmarker erhalten wurden, in jedem der Replica-Aliquots.

2.  Verfahren nach Anspruch 1, wobei jedes Aliquot in der ersten Vervielfältigungsreaktion etwa 0,1-0,9 Genome von DNA pro Vervielfältigungsreaktion umfaßt.

3.  Verfahren nach Anspruch 1 oder Anspruch 2, wobei die Kopienzahl des Testmarkers berechnet wird, indem die Anzahl an Vervielfältigungsprodukten für den Testmarker und den Referenzmarker manuell gezählt wird.

4.  Verfahren nach einem der vorangegangenen Ansprüche, wobei die Kopienzahl des Testmarkers unter Verwendung der folgenden Gleichung berechnet wird:

$$Np = N(1-e^{-z}),$$

wobei N die Anzahl an Aliquots ist, Z die durchschnittliche Anzahl an vervielfältigten Produkten pro Aliquot ist und Np die Anzahl an Aliquots ist, von denen erwartet wird, daß sie gemäß Poisson-Verteilung wenigstens ein Molekül der Nukleinsäure enthalten.

5.  Verfahren nach einem der Ansprüche 1 bis 3, wobei die Kopienzahl des Testmarkers unter Verwendung der folgenden Gleichung berechnet wird:

$$Z = -\ln(1-NP/N),$$

wobei N die Anzahl an Aliquots an Nukleinsäure ist, die für eine gegebene Sequenz getestet wurde, Np die Anzahl an Aliquots ist, die ein positives Ergebnis für die Nukleinsäure liefern (d.h. wenigstens eine Kopie der Nukleinsäure wird vervielfältigt).

6.  Verfahren nach einem der vorangegangenen Ansprüche, wobei die Vervielfältigungsreaktionen unter Verwendung von PCR durchgeführt werden.

7.  Verfahren nach einem der vorangegangenen Ansprüche, wobei die erste Vervielfältigungsreaktion unter Verwendung von Vorwärts- und Rückwärts-Primer-Paaren durchgeführt wird.

8.  Verfahren nach einem der vorangegangenen Ansprüche, wobei die zweite Vervielfältigungsreaktion unter Verwendung von Vorwärts-Internen- und Rückwärts-Primem durchgeführt wird.

9.  Verfahren nach einem der vorangegangenen Ansprüche, wobei die Konzentration von Nukleinsäure in der Probe vor dem Aliquotieren bestimmt wird, indem eine oder mehrere Nukleinsäuren, von der bzw. denen angenommen wird, daß sie in nur einer Kopie pro haploidem Genom vorliegen, in zwei oder mehreren verschiedenen Verdünnungen vervielfältigt wird bzw. werden, wobei der Anteil an Proben bei jeder Verdünnung, bei denen herausgefunden wird, daß sie für die eine oder die mehreren Nukleinsäuren positiv sind, verwendet wird, um die Schätzung der DNA-Konzentration zu verfeinern und somit die für die anschließende Analyse erforderliche Verdünnung zu bestimmen.

10. Verfahren zum Identifizieren einer oder mehrerer Veränderungen in einer Probe von Nukleinsäure, welches die Stufen umfaßt, bei denen man:

(a) die Häufigkeit der Kopienzahl einer oder mehrerer Nukleinsäuresequenzen in einer ersten Probe und einer zweiten Probe gemäß dem Verfahren nach einem der Ansprüche 1 bis 9 mißt,
(b) das Verfahren optional in allmählich höher werdenden Auflösungen für jede der Proben schrittweise wiederholt und
(c) einen oder mehrere Unterschiede in der Häufigkeit der Kopienzahl einer oder mehrerer Nukleinsäuresequenzen in den ersten und zweiten Proben identifiziert.

11. Verfahren nach Anspruch 10, wobei die Proben erkrankte und nicht erkrankte Subjekte sind oder von diesen abgeleitet sind.

12. Verfahren nach Anspruch 11, wobei die Erkrankung Krebs ist.

**13.** Verfahren nach einem der Ansprüche 10 bis 12, wobei das Verfahren anfangs bei einer Auflösung von 2 Mb durchgeführt wird und diese allmählich auf 100 Basenpaare oder weniger verringert wird.

**14.** Verfahren nach einem der Ansprüche 10 bis 13, wobei die Veränderung eine Translokation, eine Vervielfältigung, eine Duplikation oder eine Deletion ist.

**15.** Verfahren zum Diagnostizieren einer Erkrankung in einem Subjekt, welches die Stufen umfaßt, bei denen man:

(a) die Häufigkeit der Kopienzahl einer oder mehrerer Nukleinsäuresequenzen in einer Probe gemäß dem Verfahren nach einem der Ansprüche 1 bis 9 mißt,
(b) die Kopienzahl der einen oder der mehreren Nukleinsäuresequenzen mit der normalen Kopienzahl der einen oder der mehreren Nukleinsäuresequenzen vergleicht,

wobei ein Unterschied zwischen den Kopienzahlen der einen oder der mehreren Nukleinsäuresequenzen in der Probe und der normalen Kopienzahl der einen oder der mehreren Nukleinsäuresequenzen anzeigt, daß das Subjekt an der Erkrankung leidet.

**16.** Verfahren nach Anspruch 15, wobei, wenn die Kopienzahl einer oder mehrerer Nukleinsäuresequenzen in der Nukleinsäureprobe von dem Subjekt größer ist als die normale Kopienzahl, dies eine Translokation, eine Vervielfältigung oder eine Duplikation anzeigt.

**17.** Verfahren nach Anspruch 15, wobei, wenn die Kopienzahl einer oder mehrerer Nukleinsäuresequenzen in der Nukleinsäureprobe von dem Subjekt kleiner ist als die normale Kopienzahl, dies auf eine Deletion hindeutet.

**18.** Verfahren zum Klonieren einer oder mehrerer Veränderungen in einer Probe von Nukleinsäure, welches die Stufen umfaßt, bei denen man:

(a) eine oder mehrere Veränderungen in einer Probe von Nukleinsäure nach einem der Ansprüche 10 bis 14 identifiziert und
(b) die eine oder die mehreren Veränderungen kloniert.

**19.** Verfahren nach Anspruch 18, wobei die Veränderung unter Verwendung von inverser PCR-Klonierung kloniert wird.

**Revendications**

**1.** Procédé pour mesurer la fréquence du nombre de copies d'une ou plusieurs séquences d'acide nucléique dans un échantillon, comprenant les étapes suivantes :

(a) fournir une ou plusieurs aliquote(s) de l'échantillon, dans lequel chaque aliquote comprend un acide nucléique dans une quantité qui est inférieure à un génome par aliquote ;
(b) amplifier une ou plusieurs séquences d'acide nucléique dans chaque aliquote dans une première réaction d'amplification ;
(c) subdiviser un ou plusieurs des produits amplifiés en des aliquotes de réplique et effectuer une deuxième réaction d'amplification pour une ou plusieurs séquences d'acide nucléique dans chaque aliquote, dans lequel au moins une des séquences d'acide nucléique est un marqueur d'essai et au moins une des séquences d'acide nucléique est un marqueur de référence ; et
(d) calculer le nombre de copies en comparant dans chacune des aliquotes de réplique le nombre de produits amplifiés obtenus pour le marqueur d'essai avec le nombre de produits amplifiés obtenus pour le marqueur de référence.

**2.** Procédé selon la revendication 1, dans lequel chaque aliquote dans la première réaction d'amplification comprend environ 0,1 à 0,9 génomes d'ADN par réaction d'amplification.

**3.** Procédé selon la revendication 1 ou 2, dans lequel le nombre de copies du marqueur d'essai est calculé en comptant manuellement le nombre de produits d'amplification pour le marqueur d'essai et le marqueur de référence.

**4.** Procédé selon l'une quelconque des revendications précédentes, dans lequel le nombre de copies du marqueur d'essai est calculé en utilisant l'équation :

$$Np = N(1-e^{-z})$$

dans laquelle N est le nombre d'aliquotes ; Z est le nombre moyen de produits amplifiés par aliquote ; et Np est le nombre d'aliquotes qui sont supposées contenir au moins une molécule de l'acide nucléique conformément à la distribution de Poisson.

5. Procédé selon l'une quelconque des revendications 1 à 3, dans lequel le nombre de copies du marqueur d'essai est calculé en utilisant l'équation :

$$Z = -\ln(1-Np/N)$$

dans laquelle N est le nombre d'aliquotes d'acide nucléique testées pour une séquence donnée ; Np est le nombre d'aliquotes qui sont positives pour l'acide nucléique, (c'est-à-dire qu'au moins une copie de la séquence d'acide nucléique est amplifiée).

6. Procédé selon l'une quelconque des revendications précédentes, dans lequel les réactions d'amplification sont effectuées en utilisant l'amplification en chaîne par polymérase (PCR).

7. Procédé selon l'une quelconque des revendications précédentes, dans lequel la première réaction d'amplification est effectuée en utilisant des paires d'amorces sens et anti-sens.

8. Procédé selon l'une quelconque des revendications précédentes, dans lequel la deuxième réaction d'amplification est effectuée en utilisant des amorces sens internes et anti-sens.

9. Procédé selon l'une quelconque des revendications précédentes, dans lequel la concentration de l'acide nucléique dans l'échantillon avant de l'aliquoter est déterminée en amplifiant un ou plusieurs acides nucléiques considérés être présents à raison d'une seule copie par génome haploïde à deux ou davantage de dilutions différentes, dans lequel la proportion d'échantillons à chaque dilution trouvés positifs pour lesdits un ou plusieurs acides nucléiques est utilisée pour affiner l'estimation de la concentration d'ADN et donc déterminer la dilution nécessaire pour l'analyse subséquente.

10. Procédé d'identification d'une ou plusieurs altérations dans un échantillon d'acide nucléique, comprenant les étapes suivantes :

(a) mesurer la fréquence du nombre de copies d'une ou plusieurs séquences d'acide nucléique dans un premier échantillon et un deuxième échantillon conformément au procédé selon l'une quelconque des revendications 1 à 9 ;
(b) éventuellement répéter de manière itérative le procédé à des résolutions progressivement plus hautes pour chacun des échantillons ; et
(c) identifier une ou plusieurs différences dans la fréquence du nombre de copies d'une ou plusieurs séquences d'acide nucléique dans les premier et deuxième échantillons.

11. Procédé selon la revendication 10, dans lequel les échantillons sont ou sont dérivés de sujets malades et non malades.

12. Procédé selon la revendication 11, dans lequel la maladie est le cancer.

13. Procédé selon l'une quelconque des revendications 10 à 12, dans lequel le procédé est initialement mis en oeuvre à une résolution de 2 Mb décroissant progressivement jusqu'à 100 paires de bases ou moins.

**14.** Procédé selon l'une quelconque des revendications 10 à 13, dans lequel l'altération est une translocation, une amplification, une duplication ou une délétion.

**15.** Procédé pour diagnostiquer une maladie chez un sujet, comprenant les étapes suivantes :

(a) mesurer la fréquence du nombre de copies d'une ou plusieurs séquences d'acide nucléique dans un échantillon conformément au procédé selon l'une quelconque des revendications 1 à 9 ; et
(b) comparer le nombre de copies des dites une ou plusieurs séquences d'acide nucléique avec le nombre de copies normales des dites une ou plusieurs séquences d'acide nucléique ;

dans lequel une différence entre les nombres de copies des dites une ou plusieurs séquences d'acide nucléique dans l'échantillon et le nombre de copies normales des dites une ou plusieurs séquences d'acide nucléique constitue une indication que le sujet souffre de la maladie.

**16.** Procédé selon la revendication 15, dans lequel si le nombre de copies d'une ou plusieurs séquences d'acide nucléique dans l'échantillon d'acide nucléique provenant du sujet est supérieur au nombre de copies normales, ceci constitue une indication d'une translocation, d'une amplification ou d'une duplication.

**17.** Procédé selon la revendication 15, dans lequel si le nombre de copies d'une ou plusieurs séquences d'acide nucléique dans l'échantillon d'acide nucléique provenant du sujet est inférieur au nombre de copies normales, ceci constitue une indication d'une délétion.

**18.** Procédé de clonage d'une ou plusieurs altérations dans un échantillon d'acide nucléique, comprenant les étapes suivantes :

(a) identifier une ou plusieurs altérations dans un échantillon d'acide nucléique selon l'une quelconque des revendications 10 à 14 ; et
(b) cloner lesdites une ou plusieurs altérations.

**19.** Procédé selon la revendication 18, dans lequel l'altération est clonée en utilisant le clonage par PCR inverse.

FIGURE 1

24 positives (1n)          46 positives (2n)

**FIGURE 2**

**FIGURE 3**

FIGURE 4

# FIGURE 5

**A.**  XbaI  unknown sequence  chromosome 3p13  XbaI

R primer  F primer

**B.**

TATACATACATACGGATATATGTATAAAATC TAGGGAGTGAAGTAGTGGCCAAGAAAACATGCCAG

A

**C.**  Chromosome 5q21.2  Chromosome 5q21.3

5q breakpoint

**D.**  Chromosome 3p13  Chromosome 3p12.3

5p breakpoint

# FIGURE 6

A. Copy-number (genomes/aliquot)

Round 1 — ~0.25Mb intervals →

Round 2 — ~30kb intervals →

Round 3 — ~3kb intervals →

Round 4 — ~400bp intervals →

B.

FIGURE 7

SK-RC-9

Chromosome 5

**FIGURE 8**

## FIGURE 9

Marker 21  Marker 22

Marker 24  Marker 25

**FIGURE 10**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- DE 102004036285
- US 6207372 B
- US 6331395 B
- WO 9703211 A
- WO 9639154 A

### Non-patent literature cited in the description

- *Breast Cancer Res. Treat.,* vol. 18 (1), 5-14
- *Biochim. Biophys. Acta.,* vol. 1072, 33-50
- *Cancer Genet. Cytogenet.,* vol. 49, 203-217
- *Anatomic Pathology,* 1991, vol. 95 (2), 117-124
- *Cancer Res,* 1986, vol. 46, 2964-2969
- *Genomics,* 1989, vol. 4, 560
- *Science,* 1988, vol. 241, 1077
- *Proc. Nat. Acad. Sci. USA,* 1992, vol. 89, 392-396
- *Bio/Technology,* 1988, vol. 6, 1197-1202
- *Bio/Technology,* 1995, vol. 13, 563-565
- *Proc. Nat. Acad. Sci. USA,* 1990, vol. 87, 1874-1878
- *Science,* 1988, vol. 239, 487
- *Am. J. Hum. Genet.,* 1998, vol. 62 (4), 768-75
- *Am. J. Obstet. Gynecol.,* 2002, vol. 186, 117-20
- Oligonucleotides and Analogues, a Practical Approach. IRL Press at Oxford University Press, 1991
- Antisense Strategies, Annals of the New York Academy of Sciences. 1992, vol. 600
- **Milligan.** *J. Med. Chem.,* 1993, vol. 36, 1923-1937
- Antisense Research and Applications. CRC Press, 1993
- **Mata.** *Toxicol. Appl. Pharmacol.,* 1997, vol. 144, 189-197
- **Strauss-Soukup.** *Biochemistry,* 1997, vol. 36, 8692-8698
- **Samstag.** *Antisense Nucleic Acid Drug Dev,* 1996, vol. 6, 153-156
- **Devereux et al.** *Nucleic Acids Research,* 1984, vol. 12, 387
- **Atschul et al.** *J. Mol. Biol.,* 1990, 403-410
- *FEMS Microbiol Lett,* 1999, vol. 174 (2), 247-50
- *FEMS Microbiol Lett,* 1999, vol. 177 (1), 187-8
- **Simon RJ et al.** *PNAS,* 1992, vol. 89 (20), 9367-9371
- **Horwell DC.** *Trends Biotechnol.,* 1995, vol. 13 (4), 132-134
- **J. Sambrook ; E. F. Fritsch ; T. Maniatis.** Molecular Cloning: A Laboratory Manual. Cold Spring Harbor Laboratory Press, 1989, vol. 1-3
- **Ausubel, F. M. et al.** Current Protocols in Molecular Biology. John Wiley & Sons, 1995
- **B. Roe ; J. Crabtree ; A. Kahn.** DNA Isolation and Sequencing: Essential Techniques. John Wiley & Sons, 1996
- Oligonucleotide Synthesis: A Practical Approach. Irl Press, 1984
- **D. M. J. Lilley ; J. E. Dahlberg.** Methods of Enzymology: DNA Structure Part A: Synthesis and Physical Analysis of DNA Methods in Enzymology. Academic Press, 1992
- **Albertson, D.G. ; Collins, C. ; McCormick, F. ; Gray, J.W.** Chromosome aberrations in solid tumors. *Nat Genet,* 2003, vol. 34, 369-376
- **Rabbitts, T.H.** Chromosomal translocations in human cancer. *Nature,* 1994, vol. 372, 143-149
- **Huntly, B.J. ; Bench, A. ; Green, A.R.** Double jeopardy from a single translocation: deletions of the derivative chromosome 9 in chronic myeloid leukemia. *Blood,* 2003, vol. 102, 1160-1168
- **Kovacs, G. ; Szucs, S. ; De Riese, W. ; Baumgartel, H.** Specific chromosome aberration in human renal cell carcinoma. *Int. J. Cancer,* 1987, vol. 40, 171-178
- **Kovacs, G. ; Brusa, P.** Recurrent genomic rearrangements are not at the fragile sites on chromosomes 3 and 5 in human renal cell carcinomas. *Hum Genet,* 1988, vol. 80, 99-101
- **Kovacs, G. et al.** The Heidelberg classification of renal cell tumours. *J. Pathol.,* 1997, vol. 183, 131-133
- **Pinkel, D. ; Albertson, D.G.** Comparative genomic hybridization. *Annu Rev Genomics Hum Genet,* 2005, vol. 6, 331-354

- **Fiegler, H. et al.** DNA microarrays for comparative genomic hybridization based on DOP-PCR amplification of BAC and PAC clones. *Genes Chromosomes Cancer,* 2003, vol. 36, 361-374
- **Menten, B. et al.** arrayCGHbase: an analysis platform for comparative genomic hybridization microarrays. *BMC Bioinformatics,* 2005, vol. 6, 124
- **Brennan, C. et al.** High-resolution global profiling of genomic alterations with long oligonucleotide microarray. *Cancer Res,* 2004, vol. 64, 4744-4748
- **Carvalho, B. ; Ouwerkerk, E. ; Meijer, G.A. ; Ylstra, B.** High resolution microarray comparative genomic hybridisation analysis using spotted oligonucleotides. *J Clin Pathol,* 2004, vol. 57, 644-646
- **Lucito, R. et al.** Representational oligonucleotide microarray analysis: a high-resolution method to detect genome copy number variation. *Genome Res,* 2003, vol. 13, 2291-305
- **Sebat, J. et al.** Large-scale copy number polymorphism in the human genome. *Science,* 2004, vol. 305, 525-528
- **Jobanputra, V. et al.** Application of ROMA (representational oligonucleotide microarray analysis) to patients with cytogenetic rearrangements. *Genet Med,* 2005, vol. 7, 111-118
- **Zhou, W. et al.** Counting alleles reveals a connection between chromosome 18q loss and vascular invasion. *Nat Biotechnol,* 2001, vol. 19, 78-81
- **Zhao, X. et al.** Homozygous deletions and chromosome amplifications in human lung carcinomas revealed by single nucleotide polymorphism array analysis. *Cancer Res,* 2005, vol. 65, 5561-5570
- **Heim, S. ; Mitelman, F.** Cytogenetics of solid tumours. *Recent Advances in Histopathology,* 1991, 37-66
- **Hoglund, M. et al.** Dissecting karyotypic patterns in renal cell carcinoma: an analysis of the accumulated cytogenetic data. *Cancer Genet Cytogenet,* 2004, vol. 153, 1-9
- **Dear, P.H. ; Cook, P.R.** Happy mapping: a proposal for linkage mapping the human genome. *Nucleic Acids Res,* 1989, vol. 17, 6795-6807
- **Ebert, T. ; Bander, N.H. ; Finstad, C.L. ; Ramsawak, R.D. ; Old, L.J.** Establishment and characterisation of human renal cancer and normal kidney cell lines. *Cancer Res.,* 1990, vol. 50, 5531-5536
- **Suzuki, T. et al.** New genes involved in cancer identified by retroviral tagging. *Nat Genet,* 2002, vol. 32, 166-174
- **Cohen, H.T. ; McGovern, F.J.** Renal-cell carcinoma. *N Engl J Med,* 2005, vol. 353, 2477-2490
- **Daruwala, R.S. et al.** A versatile statistical analysis algorithm to detect genome copy number variation. *Proc Natl Acad Sci USA,* 2004, vol. 101, 16292-16297
- **LeFranc, M.-P. ; Forster, A. ; Baer, R. ; Stinson, M.A. ; Rabbitts, T.H.** Diversity and rearrangement of the human T cell rearranging $\gamma$ genes: Nine germ-line variable genes belonging to two subgroups. *Cell,* 1986, vol. 45, 237-246
- **Feinberg, A.P. ; Vogelstein, B.A.** A technique for radiolabelling DNA restriction endonuclease fragments to high specific activity. *Anal. Biochem.,* 1983, vol. 132, 6-13